# FASCICULE DE BREVET EUROPEEN

(11) **EP 0 573 361 B1**
(45) Date de publication et mention de la délivrance du brevet: **11.12.1996**
(21) Numéro de dépôt: 93401418.4
(22) Date de dépôt: 03.06.1993
(51) Int. Cl.: C07C 69/743, C07C 69/757, C07C 255/39, C07C 67/00, C07C 67/307

(54) **Procédé de préparation d'esters de l'acide 2,2-diméthyl 3-((Z)-2-(alcoxycarbonyl)ethenyl cyclopropane carboxylique et intermédiaires**
Verfahren zur Herstellung von 2,2,-Dimethyl-3-((Z)-2-(Alkoxycarbonyl)Ethenyl)Cyclopropancarbonsäureestern und Zwischenprodukte
Process for the preparation of esters of 2,2-dimethyl-3-((Z)-2-(alkoxycarbonyl)ethenyl)cyclopropanecarboxylic acid and intermediates

(30) Priorité: 04.06.1992 FR 9206772
(43) Date de publication de la demande: 08.12.1993
(73) Titulaire: ROUSSEL UCLAF, 93230 Romainville (FR)
(72) Inventeur: Babin, Didier, F-78180 Montigny (FR); Bahtnagar, Neerja, F-91600 Savigny sur Orge (FR); Brion, Francis, F-93220 Gagny (FR); Colladant, Colette, F-93110 Rosny sous Bois (FR)
(74) Mandataire: Vieillefosse, Jean-Claude

(56) Documents cités:
- FR-A- 2 520 735
- GB-A- 2 102 408
- TETRAHEDRON LETTERS, vol. 27, no. 35, 1986, Oxford, GB, pages 4119 - 4120, T. A. ENGLER et al, "Stereoselective preparation of methyl (Z)-cinnamates by Favorskii rearrangement"
- ORGANIC SYNTHESES, vol. 53, 1973, New York, US, pages 123 - 127, C. RAPPE, "Cis-alpha,beta-unsaturated acids: Isocrotonic acid"

## Description

La présente invention concerne un procédé de préparation d'esters de l'acide 2,2-diméthyl 3-[(Z) 1-propényl] cyclopropane carboxylique et des intermédiaires.

L'invention a ainsi pour objet un procédé de préparation des composés de formule (I) : 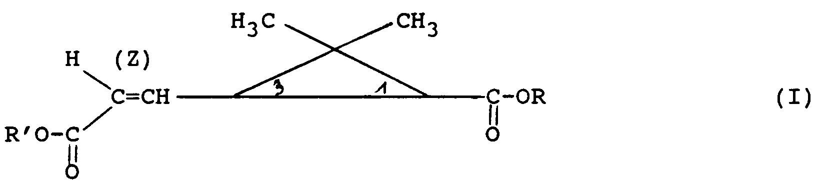
dans laquelle R représente ou bien un reste d'ester clivable ou bien un reste d'ester connu dans la chimie des pyréthrinoïdes, à savoir :
a) soit un radical benzyle éventuellement substitué sur les sommets aromatiques par un ou plusieurs radicaux choisis dans le groupe constitué par les radicaux alkyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylènedioxy et les atomes d'halogène,
b) soit un groupement 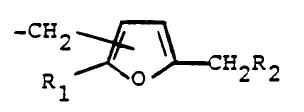 dans lequel le substituant R₁ représente un atome d'hydrogène ou un radical méthyle et le substituant R₂ un aryle monocyclique ou un groupement -C≡CH,
c) soit un groupement 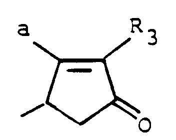 dans lequel a représente un atome d'hydrogène ou un radical méthyle et R₃ représente un radical organique aliphatique comportant de 2 à 6 atomes de carbone et une ou plusieurs insaturations carbone-carbone,
d) soit un groupement 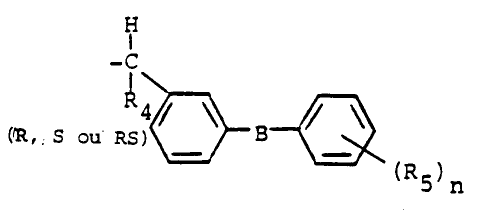 dans lequel B représente un atome d'oxygène ou de soufre ou un groupement 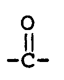 ou -CH₂-, R₄ représente un atome d'hydrogène, un atome de chlore, de brome ou d'iode, un radical -C≡N, un radical méthyle, un radical -CONH₂, un radical -CSNH₂ ou un radical -C≡CH, R₅ représente un atome d'halogène ou un radical méthyle et n représente un nombre égal à 0, 1 ou 2,
e) soit un groupement 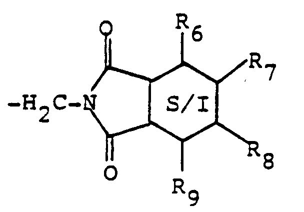 dans lequel les substituants R₆, R₇, R₈ et R₉ représentent un atome d'hydrogène, un atome de chlore ou un radical méthyle et dans lequel S/I symbolise un cycle aromatique ou un cycle analogue dihydro, tétrahydro ou hexahydro,
f) soit un groupement (succimido ou maléimido) méthylène,
g) soit un groupement 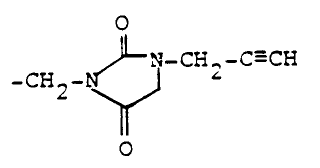
h) soit un groupement 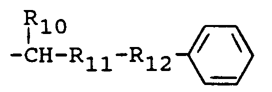 dans lequel R₁₀ représente un atome d'hydrogène ou un radical CN, R₁₂ représente un radical -CH₂- ou un atome d'oxygène, R₁₁ représente un radical thiazolyle ou thiadiazolyle dont la liaison avec 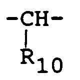 peut se trouver à l'une quelconque des positions disponibles, R₁₂ étant lié à R₁₁ par l'atome de carbone compris entre l'atome de soufre et un atome d'azote.
i) soit un groupement 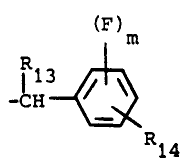 dans lequel R₁₃ représente un atome d'hydrogène ou un radical -CN ou -C≡CH, R₁₄ représente un radical trifluorométhyle ou un radical alkyle, alcényle ou alcynyle renfermant au maximum 6 atomes de carbone et m représente un nombre égal à 1, 2, 3 ou 4,
j) soit un groupement 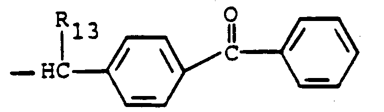 dans lequel R₁₃ est défini comme ci-dessus,
k) soit un groupement 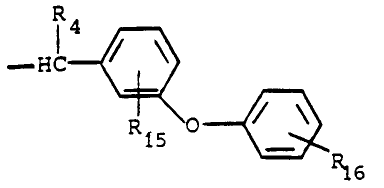 dans lequel R₄ est défini comme ci-dessus, R₁₅ représente un atome de fluor, de chlore ou de brome et R₁₆ représente un atome d'hydrogène, de fluor, de chlore ou de brome,
l) soit un groupement 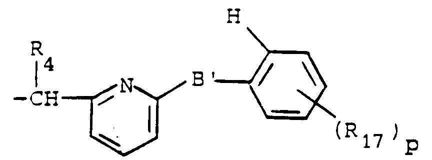 dans lequel R₄ est défini comme ci-dessus, chacun des R₁₇ représente indépendamment un groupement alkyle renfermant de 1 à 4 atomes de carbone, alcoxy renfermant de 1 à 4 atomes de carbone, alkylthio renfermant de 1 à 4 atomes de carbone, alkylsulfonyl renfermant de 1 à 4 atomes de carbone, trifluorométhyle, 3,4-méthylènedioxy, chloro, fluoro ou bromo, p représente un nombre égal à 0, 1 ou 2 et B' représente un atome d'oxygène ou un atome de soufre,
m) soit un groupement 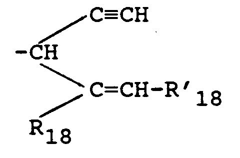 dans lequel R₁₈ représente un atome de fluor ou un radical méthyle et R'₁₈ représente un radical méthyle, éthyle ou propargyle,
n) soit un groupement 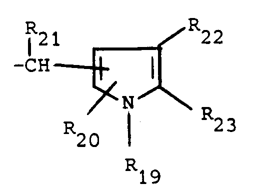 dans laquelle R₂₁ représente un atome d'hydrogène, un groupement -C≡N, -C≡CH, -CF₃ ou un radical alkyle renfermant de 1 à 3 atomes de carbone, R₂₀, R₂₂ et R₂₃ identiques ou différents les uns des autres, représentent un atome d'hydrogène, un atome d'halogène, un radical alkyle renfermant jusqu'à 18 atomes de carbone, un radical aryle renfermant jusqu'à 14 atomes de carbone, un radical aralkyle renfermant jusqu'à 18 atomes de carbone, un radical cyano, un radical -CF₃, un radical -CO₂-alkyle renfermant jusqu'à 8 atomes de carbone, un radical NO₂, un radical alcoxy renfermant jusqu'à 8 atomes de carbone, un radical 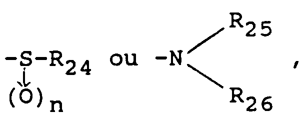 n étant égal à 0, 1 ou 2 et les radicaux R₂₄, R₂₅ et R₂₆ représentant un radical alkyle renfermant de 1 à 8 atomes de carbone, les radicaux R₂₂ et R₂₃ pouvant former un homocycle carboné saturé ou insaturé renfermant jusqu'à 8 atomes de carbone et R₁₉ représente
   - ou bien un radical 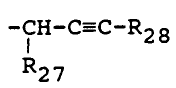 dans lequel R₂₇ et R₂₈, identiques ou différents l'un de l'autre, représentent un atome d'hydrogène, un atome d'halogène, un radical alkyle renfermant de 1 à 8 atomes de carbone ou un radical aryle renfermant jusqu'à 14 atomes de carbone
   - ou bien un radical 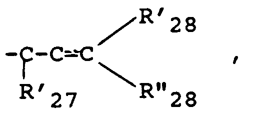 dans lequel R'₂₇, R'₂₈ et R''₂₈, identiques ou différents les uns des autres, représentent l'une des valeurs indiquées ci-dessus pour R₂₇ et R₂₈, les traits pointillés représentent une seconde liaison éventuelle,
   - ou bien un radical 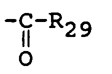 dans lequel R₂₉ peut avoir les valeurs indiquées précédemment pour R₂₂ et R₂₃, à l'exception d'halogène, cyano, NO₂, 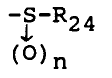 dans lequel n est égal à 1 ou 2 et 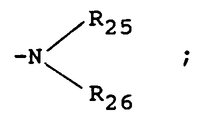
   - ou bien un radical 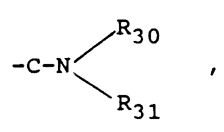 dans lequel R₃₀ et R₃₁, identiques ou différents l'un de l'autre, représentent un atome d'hydrogène, un radical alkyle renfermant de 1 à 18 atomes de carbone, un radical aryle renfermant jusqu'à 14 atomes de carbone, un radical aralkyle renfermant jusqu'à 18 atomes de carbone, un radical CF₃, un radical CO₂-alkyle renfermant jusqu'à 8 atomes de carbone ou un radical alcoxy renfermant jusqu'à 8 atomes de carbone,
o) soit un groupement 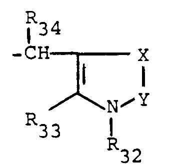 dans laquelle X représente un atome de soufre ou d'oxygène, Y représente un groupe >C=O, >C=S ou >CH₂, R₃₂ représente un atome d'hydrogène, un radical alkyle, linéaire, ramifié ou cyclique, saturé ou insaturé, renfermant jusqu'à 8 atomes de carbone, éventuellement substitué par un ou plusieurs halogènes, ou un radical aryle renfermant jusqu'à 14 atomes de carbone, R₃₃ représente un atome d'hydrogène, un radical alkyle linéaire, ramifié ou cyclique, saturé ou insaturé, renfermant jusqu'à 8 atomes de carbone, éventuellement substitué par un ou plusieurs halogènes, ou R₃₃ représente un radical aryle renfermant jusqu'à 14 atomes de carbone, un radical -CF₃, -NO₂, -C≡N, un atome d'halogène, un radical alcoxyle renfermant jusqu'à 8 atomes de carbone ou un radical -CO₂-alkyle renfermant jusqu'à 8 atomes de carbone, R₃₄ représente un atome d'hydrogène, un radical alkyle renfermant de 1 à 3 atomes de carbone, un radical -C≡CH,
p) soit un groupement 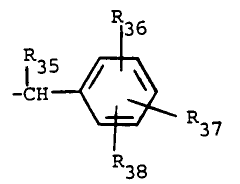 dans lequel R₃₅ représente un atome d'hydrogène, un radical alkyle renfermant de 1 à 3 atomes de carbone, un radical -C≡N, -C≡CH ou CF₃, R₃₆ et R₃₈, identiques ou différents l'un de l'autre, représentent un atome d'hydrogène, un radical alkyle renfermant de 1 à 3 atomes de carbone éventuellement substitué par un ou plusieurs atomes d'halogène, un radical alcényle renfermant de 2 à 4 atomes de carbone ou un atome d'halogène et R₃₇ représente un radical phényle éventuellement substitué par un ou plusieurs radicaux alkyle renfermant de 1 à 3 atomes de carbone ou par un ou plusieurs atomes d'halogène, R' représente un atome d'hydrogène, un radical alkyle renfermant de 1 à 18 atomes de carbone, linéaire, ramifié, saturé ou insaturé éventuellement substitué par un ou plusieurs groupements fonctionnels, identiques ou différents, ou bien R' représente un radical cycloaliphatique comportant de 3 à 7 atomes de carbone éventuellement substitué par un ou plusieurs groupements fonctionnels identiques ou différents, ou bien R' représente un groupement aryle renfermant de 6 à 14 atomes de carbone éventuellement substitué par un ou plusieurs groupements fonctionnels identiques ou différents, ou bien R' représente un radical hétérocyclique éventuellement substitué par un ou plusieurs groupements fonctionnels identiques ou différents, sous forme de mélanges d'isomères ou d'isomères séparés au niveau du cycle cyclopropane, caractérisé en ce que l'on traite un ester de formule (II) : 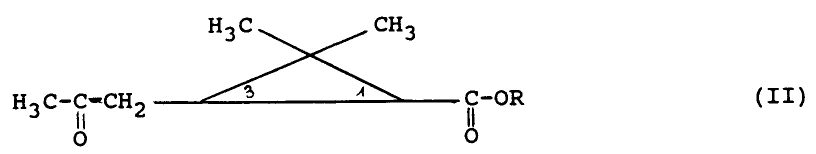 dans laquelle R est défini comme ci-dessus, par un agent d'halogénation, pour obtenir un composé de formule (III) : 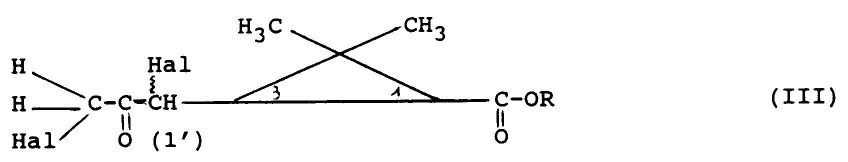 dans laquelle R est défini comme ci-dessus, et Hal représente un atome d'halogène, se présentant sous la forme d'un mélange d'isomères au niveau de l'atome de carbone en position 1', que l'on traite par un agent basique en présence d'un composé de formule R'-OH, dans laquelle R' est défini comme ci-dessus, pour obtenir le composé de formule (I) attendu.

Lorsque R représente un reste d'ester clivable, il peut s'agir de tous restes connus de l'homme du métier, notamment d'un radical alkyle renfermant de 1 à 18 atomes de carbone, tel que méthyle, éthyle, n-propyle, isopropyle, n-butyle, isobutyle ou de préférence, terbutyle, ou d'un radical alkyle tel que défini ci-dessus, substitué par un ou plusieurs 5 atomes d'halogène, de préférence chlore ou brome, tel que bromo ou chlorométhyle, mono, di ou tri bromo ou chloro éthyle ; il peut encore s'agir notamment d'un radical alkyle silylé dans lequel alkyle renferme de 1 à 4 atomes de carbone, tel que trialkylsilyle méthyle, par exemple terbutyldiméthylsilylméthyle ou arylalkylsilylméthyle tel que diphénylterbutylsilylméthyle ; il peut encore s'agir notamment d'un radical alkyle substitué par un groupement O-alkyle, O-aryle ou O-aralkyle, alkyle renfermant de préférence de 1 à 4 atomes de carbone, aryle renfermant de 6 à 14 atomes de carbone et représentant de préférence un radical phényle ou tolyle et aralkyle représentant de préférence un radical benzyle ou phénéthyle.

Il convient seulement de noter que le reste d'ester ci-dessus est tel qu'il soit clivable en milieu neutre ou acide.

Lorsque R représente un radical benzyle substitué par un ou plusieurs radicaux alkyle, il s'agit de préférence des radicaux méthyle, éthyle, propyle ou isopropyle.

Lorsque R représente un radical benzyle substitué par un ou plusieurs radicaux alcényle, il s'agit de préférence de radicaux vinyle, allyle, 2-méthylallyle ou isobutényle.

Lorsque R représente un radical benzyle substitué par un ou plusieurs radicaux alcényloxy, il s'agit de préférence de radicaux vinyloxy, allyloxy, 2-méthylallyloxy ou isobutényloxy.

Lorsque R représente un radical benzyle substitué par un ou plusieurs radicaux alcadiényle, il s'agit de préférence d'un radical butadiényle ou pentadiényle.

Lorsque R représente un radical benzyle substitué par un ou plusieurs atomes d'halogène, il s'agit de préférence d'atomes de chlore, de brome ou de fluor.

Lorsque R₂ représente un radical aryle, il s'agit de préférence du radical phényle et le groupement R est alors de préférence le groupement 5-benzyl 3-furylméthyle.

R₃ peut représenter notamment un radical -CH₂-CH=CH₂, -CH₂-CH=CH-CH₃, -CH₂-CH=CH-CH=CH₂, -CH₂-CH=CH-CH₂-CH₃ ou -CH₂-C≡CH.

B représente notamment un atome d'oxygène et le groupement correspondant est alors de préférence 3-phénoxybenzyle, α-cyano 3-phénoxybenzyle, α-éthynyl 3-phénoxybenzyle, 1-(3-phénoxyphényl) éthyle ou α-thioamido 3-phénoxybenzyle. B représente encore notamment un groupe -CO- et le groupement correspondant est alors de préférence 3-benzoylbenzyle.

Lorsque R₁₄ représente un radical alkyle, il s'agit de préférence d'un radical méthyle, éthyle, propyle, linéaire ou ramifié ou butyle linéaire ou ramifié.

Lorsque R₁₄ représente un radical alcényle, il s'agit d'un radical vinyle, allyle, butényle, pentényle ou hexényle.

Lorsque R₁₄ représente un radical alcynyle, il s'agit de préférence d'un radical éthynyle, propargyle ou butynyle.

Lorsque R₁₇ représente un radical alkyle, il s'agit d'un radical méthyle, éthyle, propyle linéaire ou ramifié ou butyle linéaire ou ramifié.

Lorsque R₁₇ représente un radical alcoxy, il s'agit d'un radical méthoxy, éthoxy, propoxy linéaire ou ramifié ou butoxy linéaire ou ramifié.

Lorsque R₁₇ représente un radical alkylthio, il s'agit d'un radical méthylthio, éthylthio, propylthio linéaire ou ramifié ou butylthio linéaire ou ramifié.

Lorsque R₁₇ représente un radical alkylsulfonyle, il s'agit d'un radical correspondant à l'un quelconque des radicaux alkylthio ci-dessus.

Lorsque R₂₁ représente un radical alkyle, il s'agit d'un radical méthyle, éthyle, propyle ou isopropyle et de préférence du radical méthyle.

Lorsqu'un ou plusieurs radicaux R₂₀, R₂₂ et R₂₃ ou contenus dans les définitions de R₁₉, R₂₀, R₂₂ et R₂₃ représentent un atome d'halogène, il s'agit de préférence d'un atome de fluor, de chlore ou de brome.

Lorsqu'un ou plusieurs radicaux R₂₀, R₂₂ et R₂₃ ou contenus dans les définitions de R₁₉, R₂₀, R₂₂ et R₂₃ représentent un radical alkyle, il s'agit de préférence d'un radical méthyle, éthyle, propyle, isopropyle, butyle, isobutyle ou n-pentyle.

Lorsqu'un ou plusieurs radicaux R₂₀, R₂₂ et R₂₃ ou contenus dans les définitions de R₁₉, R₂₀, R₂₂ et R₂₃ repré-sentent un radical aryle, il s'agit de préférence du radical phényle et celui-ci peut être substitué notamment par un radical alkyle ou alcoxy renfermant de 1 à 8 atomes de carbone, ou par un radical nitro, trifluorométhyle, hydroxy, halogéno ou amino.

Lorsqu'un ou plusieurs radicaux R₂₀, R₂₂ et R₂₃ ou contenus dans les définitions de R₁₉, R₂₀, R₂₂ et R₂₃ repré-sentent un radical aralkyle, il s'agit de préférence du radical benzyle.

Lorsqu'un ou plusieurs radicaux R₂₀, R₂₂ et R₂₃ ou contenus dans les définitions de R₁₉, R₂₀, R₂₂ et R₂₃ représentent un radical -CO₂-alkyle ou un radical alcoxy, on entend de préférence par alkyle, un radical méthyle, éthyle, propyle ou isopropyle et par alcoxy, un radical méthoxy, éthoxy, propoxy ou isopropoxy.

Lorsque R₃₂ ou R₃₃ représente un radical alkyle linéaire, ramifié ou cyclique, saturé ou insaturé, il s'agit de préférence d'un radical méthyle, éthyle, n-propyle, isopropyle, cyclopropyle, cyclopropylméthyle, butyle, isobutyle, tertbutyle, cyclobutyle, n-pentyle, cyclopentyle, n-hexyle ou cyclohexyle, ou d'un radical allyle, propargyle ou butynyle.

Lorsque ces radicaux sont substitués par un ou plusieurs atomes d'halogène, on entend par halogène, le fluor, le chlore, le brome ou l'iode.

Lorsque R₃₂ ou R₃₃ représente un radical aryle, il s'agit de préférence du radical phényle.

Lorsque R₃₃ représente un atome d'halogène, il s'agit de préférence d'un atome de fluor, de chlore ou de brome.

Lorsque R₃₃ représente un radical alcoxy, il s'agit de préférence d'un radical méthoxy, éthoxy, propoxy ou isopropoxy.

Lorsque R₃₃ représente un radical CO₂-alkyle, par alkyle on entend de préférence un radical méthyle, éthyle, propyle linéaire ou ramifié ou butyle linéaire ou ramifié.

Lorsque R₃₄ représente un radical alkyle, il s'agit de préférence d'un radical méthyle.

Lorsque R₃₃ représente un radical alkyle, il s'agit de préférence d'un radical méthyle.

Lorsque R₃₆ et/ou R₃₈ représentent un radical alkyle, il s'agit de préférence d'un radical méthyle ou éthyle.

Lorsque le radical alkyle est substitué par un ou plusieurs atomes d'halogène, on entend de préférence par halogène, le fluor, le chlore ou le brome.

Lorsque R₃₆ et/ou R₃₈ représentent un radical alcényle, il s'agit de préférence d'un radical vinyle ou allyle.

Lorsque R₃₆ et/ou R₃₈ représentent un atome d'halogène, il s'agit de préférence d'un atome de fluor, de chlore ou de brome.

Lorsque R₃₇ représente un radical phényle substitué par un radical alkyle ou par un atome d'halogène, on entend de préférence par alkyle et halogène les radicaux correspondants mentionnés plus haut pour R₃₆ et R₃₈.

Lorsque R' représente un radical alkyle linéaire ou ramifié, on entend par alkyle, par exemple, un radical méthyle, éthyle, propyle linéaire ou ramifié, butyle linéaire ou ramifié, hexyle linéaire ou ramifié, décyle linéaire ou ramifié, tétradécyle linéaire ou ramifié, octadécyle linéaire ou ramifié.

Lorsque R' représente un radical alkyle linéaire ou ramifié, on entend par alkyle insaturé, un radical éthényle, propényle, butényle linéaire ou ramifié, hexényle linéaire ou ramifié, décényle linéaire ou ramifié, tétradécényle linéaire ou ramifié, octadécényle linéaire ou ramifié, ou encore des radicaux aliphatiques insaturés comportant deux ou plusieurs doubles liaisons.

Lorsque R' représente un radical alkyle, substitué par un ou plusieurs groupements fonctionnels, on entend de préférence par groupement fonctionnel, un atome d'halogène, un groupement OH ou SH, un groupement ORₐ ou SRₐ dans lesquels Rₐ représente un radical alkyle renfermant de 1 à 8 atomes de carbone, un groupement NO₂ ou 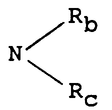
dans lequel R_{b} et R_{c}, identiques ou différents, représentent un atome d'hydrogène, ou un radical alkyle renfermant de 1 à 8 atomes de carbone, un groupement C≡N, SO₃H ou PO₄H₂ ou un groupement COalc₁, SO₂alc₂ ou SO₃alc₃ dans lesquels alc₁, alc₂ et alc₃ représentent des radicaux alkyle renfermant de 1 à 18 atomes de carbone.

R' peut représenter également un radical alkyle substitué par un radical aryle comme par exemple le radical benzyle ou le radical phénéthyle, lui-même éventuellement substitué par un ou plusieurs groupements OH, Oalc ou alc renfermant de 1 à 8 atomes de carbone, par un ou plusieurs groupements CF₃, OCF₃, SCF₃ ou par un groupement (G) :

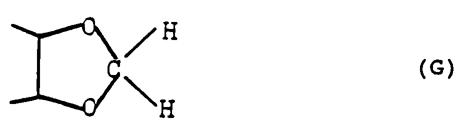

R' peut représenter également un radical alkyle substitué sur 2 carbones adjacents par un groupement (G₁) : 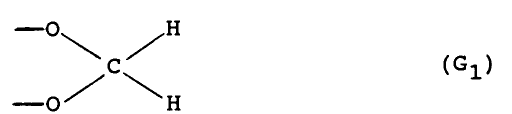
ou substitué par un groupement

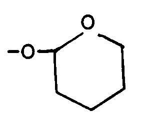

Lorsque R' représente un radical alkyle substitué par un ou plusieurs groupements fonctionnels, on peut citer comme valeurs préférées de R' les radicaux :
-(CH₂)ₙ-CHal₃ dans lequel n est un entier de 1 à 8 et Hal un atome d'halogène, par exemple le radical -CH₂-CCl₃, -CH₂-CF₃, -CH₂-CH₂-CCl₃ ou -CH₂-CH₂-CF₃,
-(CH₂)ₙ₁-CHHal₂ dans lequel Hal est défini comme ci-dessus et n1 est un nombre de 0 à 8, par exemple le radical -CH₂-CHCl₂, -CH₂-CHF₂ ou -CHF₂,
-(CH₂)ₙ-CH₂Hal dans lequel n et Hal sont définis comme ci-dessus, par exemple le radical -CH₂-CH₂Cl ou -CH₂-CH₂F,
-C (CHal₃)₃ dans lequel Hal est défini comme ci-dessus, par exemple le radical -C(CF₃)₃ ou 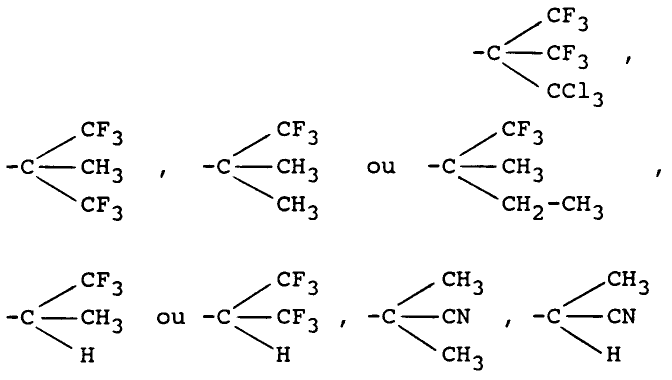 ou (CH₂)ₙ-CN
dans lequel n est défini comme précédemment, 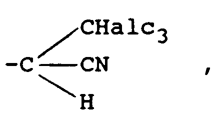
dans lequel Hal est défini comme précédemment, par exemple le radical 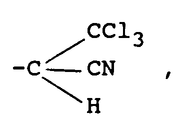
-(CH₂)ₙ-OR_{d} dans lequel n est défini comme précédemment et R_{d} représente un atome d'hydrogène ou un radical alkyle linéaire ou ramifié, comportant de 1 à 8 atomes de carbone, par exemple le radical -CH₂-OCH₃, -CH₂-CH₂-O-CH₃, -CH₂-CH₂-O-CH₂-CH₃ ou -CH₂-CH₂-OH, 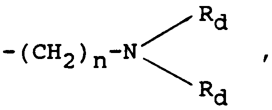
dans lequel n et R_{d} sont définis comme précédemment et les deux radicaux R_{d} peuvent être différents entre eux, par exemple le radical 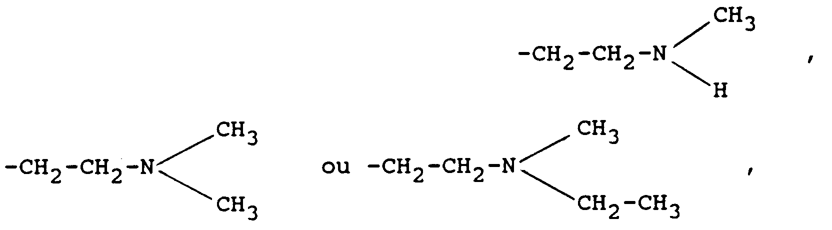 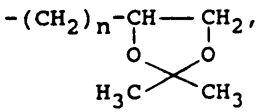
dans lequel n est défini comme précédemment, par exemple le radical 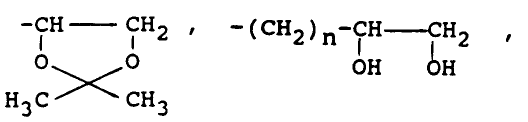
dans lequel n est défini comme précédemment, par exemple le radical 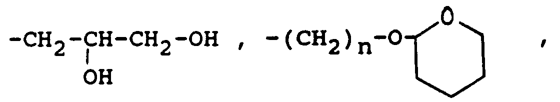
dans lequel n est défini comme précédemment, par exemple le radical 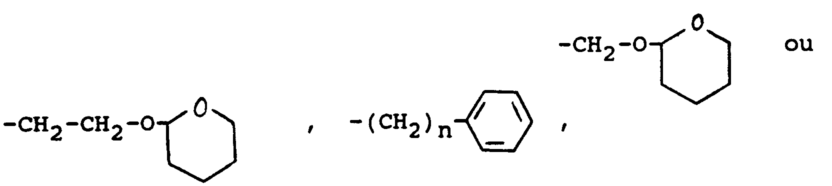
dans lequel n est défini comme précédemment, par exemple le radical benzyle ou phénéthyle, 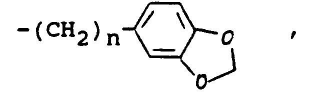
dans lequel n est défini comme précédemment, par exemple le radical

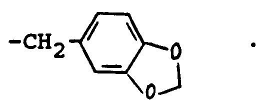

Lorsque R' représente un radical cycloaliphatique comportant de 3 à 7 atomes de carbone, il s'agit d'un radical cyclopropyle, cyclobutyle, cyclopentyle, cyclohexyle ou cycloheptyle, éventuellement relié à l'atome d'oxygène par un radical alkyle renfermant de 1 à 3 atomes de carbone, notamment par un radical méthyle.

Lorsque R' représente un radical cycloaliphatique comportant de 3 à 7 atomes de carbone, substitué par un ou plusieurs groupements fonctionnels, on entend, de préférence, par groupement fonctionnel, un atome d'halogène, un radical alkyle comportant de 1 à 6 atomes de carbone, un radical alcoxy comportant de 1 à 6 atomes de carbone, un groupement NO₂ .

Lorsque R' représente un radical aryle éventuellement substitué, il s'agit de préférence du radical phényle ou du radical phényle substitué par un ou plusieurs groupements OH, Oalc ou alc, alc représentant un radical alkyle renfermant de 1 à 8 atomes de carbone, ou par un groupement CF₃, OCF₃ ou SCF₃ ou par un ou plusieurs atomes d'halogène.

Lorsque R' représente un radical hétérocyclique éventuellement substitué, il s'agit de préférence du radical pyridyle, furyle, thiophényle, oxazolyle ou thiazolyle, éventuellement substitué par un ou plusieurs des groupements ci-dessus.

Le procédé de l'invention est d'une généralité remarquable. Il permet d'obtenir aussi bien des esters clivables, donc des esters intermédiaires, transformables en esters biologiquement actifs par des procédés classiques de transestérification ou d'hydrolyse suivie d'un estérification, que directement des esters biologiquement actifs.

Il est en outre d'une sélectivité remarquable, dans la mesure où il conduit aux composés de formule (I) de configuration (Z), c'est-à-dire à la forme biologiquement active de ces composés.

L'invention a notamment pour objet un procédé tel que défini plus haut, caractérisé en ce que l'agent d'halogénation est un agent de chloration ou de bromation, ce dernier étant plus particulièrement préféré.

Parmi les agents d'halogénation selon l'invention, on peut citer le brome utilisé seul ou sur support polymère, le chlore, les N-bromo et N-chloro succinimides et les N-bromo et N-chloro acétamides, le perbromure de pyridinium, le perbromure d'hydrobromure de pyridinium, le perchlorure d'hydrochlorure de pyridinium, le perbromure de phényltriméthylammonium, le perbromure de 2-carboxyéthyltriphénylphosphonium, le tribromure de 2-hydropyrrolidone, les bromure et chlorure cuivriques, le 5,5-dibromo 2,2-diméthyl 4,6-dioxo 1,3-dioxanne, les tri et penta bromure et chlorure de phosphore, les mélanges triméthylbromo et triméthylchloro silane-DMSO-amine tertiaire, le chlorure ferrique. Parmi ceux-ci, on peut citer plus particulièrement le brome, le chlore, les N-bromo et N-chloro succinimides et acétamides, les perbromure et perchlorure de pyridinium, les perbromure et perchlorure d'hydrobromure et hydrochlorure de pyridinium.

La réaction d'halogénation est mise en oeuvre au sein d'un solvant qui peut être un solvant halogéné, notamment le chlorure de méthylène, le chloroforme, le tétrachlorure de carbone, le dichloroéthane, ou un mélange de ceux-ci, un éther tel que le tétrahydrofuranne, le diméthoxyéthane, le méthyl terbutyléther ou le dioxanne, un solvant aromatique tel que le benzène, le toluène, le xylène ou un solvant saturé correspondant, notamment le cyclohexane, un alcool tel que le méthanol ou l'éthanol, l'acétate d'éthyle ou encore le diméthylformamide ou le diméthylsulfoxyde.

Il est avantageux de limiter au maximum la formation de dérivés mono et trihalogénés, à côté du dérivé (III) attendu et l'invention a ainsi notamment pour objet un procédé caractérisé en ce que l'on utilise environ 2 équivalents de l'agent d'halogénation.

Dans des conditions préférentielles de mise en oeuvre de l'invention, l'agent basique utilisé est choisi dans le groupe constitué par les hydrures, les alcoolates, les amidures, les carbonates alcalins et alcalino-terreux, plus particulièrement de sodium ou de potassium, les amines tertiaires, notamment la triéthylamine, la pyridine ou la diméthylaminopyridine.

On opère au sein d'un solvant qui est soit le composé de formule R'-OH, soit un mélange de ce composé avec un cosolvant approprié. Celui-ci peut être, en particulier, un solvant halogéné, un éther, un solvant aromatique ou un solvant saturé correspondant, tel que ceux qui ont été mentionnés plus haut, ou encore le diméthylformamide ou le diméthylsulfoxyde.

On opère à une température compatible avec le solvant utilisé, pouvant aller, selon le cas, de -78°C à +40°C environ.

L'invention a notamment pour objet un procédé tel que défini précédemment, caractérisé en ce que R représente un reste d'ester clivable en milieu neutre ou acide, choisi dans le groupe constitué par les radicaux alkyle, linéaires ou ramifiés renfermant de 1 à 18 atomes de carbone, les radicaux alkyles linéaires ou ramifiés renfermant de 1 à 18 atomes de carbone substitués par un ou plusieurs atomes d'halogène, les radicaux alkyles renfermant de 1 à 4 atomes de carbone, substitués par un groupement silylé et les radicaux alkyles renfermant de 1 à 4 atomes de carbone substitués par un groupement O-alkyle, O-aryle ou O-aralkyle, alkyle renfermant de 1 à 4 atomes de carbone et aryle renfermant de 6 à 14 atomes de carbone.

L'invention a plus particulièrement pour objet un procédé tel que défini précédemment, caractérisé en ce que R représente un reste d'ester clivable en milieu neutre ou acide, choisi dans le groupe constitué par les radicaux alkyles renfermant de 1 à 4 atomes de carbone, les radicaux alkyles renfermant de 1 à 4 atomes de carbone substitué par un ou plusieurs atomes de chlore ou de brome, les radicaux alkyles renfermant de 1 à 4 atomes de carbone substitués par un groupement alkylsilyle et les radicaux alkyles renfermant de 1 à 4 atomes de carbone substitués par un groupement O-alkyle, O-aryle ou O-aralkyle, tel que défini plus haut.

L'invention a tout particulièrement pour objet un procédé tel que défini précédemment, caractérisé en ce que R représente un radical alkyle renfermant de 1 à 4 atomes de carbone, de préférence terbutyle, ou un radical méthyle ou éthyle substitué par un ou plusieurs atomes de chlore ou de brome, de préférence un radical bromo ou chloro méthyle ou un radical mono, di ou tribromo ou chloroéthyle.

L'invention a encore notamment pour objet un procédé tel que défini précédemment, caractérisé en ce que R représente un reste d'ester choisi dans le groupe constitué par :
- le radical benzyle substitué par un ou plusieurs atomes d'halogène,
- les radicaux de formule : 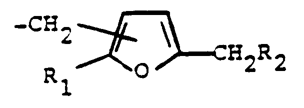 dans laquelle R₁ et R₂ sont définis comme précédemment,
- les radicaux de formule : 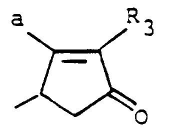 dans laquelle a et R₃ sont définis comme précédemment,
- les radicaux de formule : 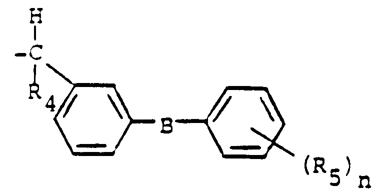 dans laquelle R₄, R₅ et n sont définis comme précédemment,
- les radicaux de formule : 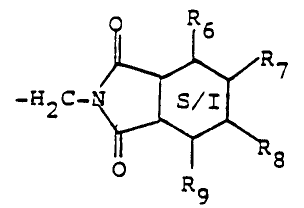 dans laquelle R₆, R₇, R₈, R₉ et S/I sont définis comme précédemment,
- les radicaux de formule : 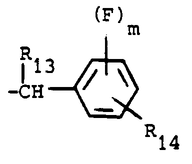 dans laquelle R₁₃, R₁₄ et m sont définis comme précédemment,
- les radicaux de formule : 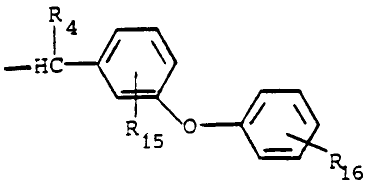 dans laquelle R₄, R₁₅ et R₁₆ sont définis comme précédemment,
- les radicaux de formule : 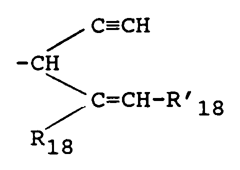 dans laquelle R₁₈ et R'₁₈ sont définis comme précédemment,
- les radicaux de formule : 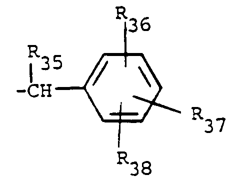 dans laquelle R₃₅, R₃₆, R₃₇ et R₃₈ sont définis comme précédemment.

L'invention a plus particulièrement pour objet un procédé tel que défini précédemment, caractérisé en ce que R représente un reste d'ester choisi dans le groupe constitué par :
- le radical benzyle substitué par 1 à 5 atomes de fluor,
- les radicaux de formule : 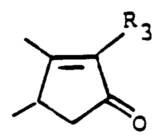 dans laquelle R₃ représente un radical -CH₂-CH-CH₂ ou -CH₂-C≡CH,
- les radicaux de formule : 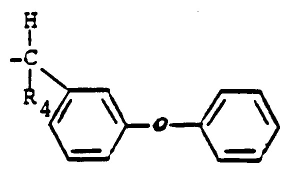 dans laquelle R₄ est défini comme précédemment,
- le radical de formule : 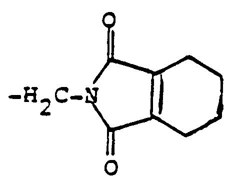
- les radicaux de formule : 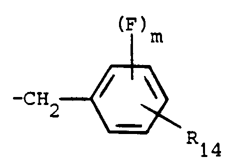 dans laquelle R₁₄ et m sont définis comme précédemment,
- les radicaux de formule : 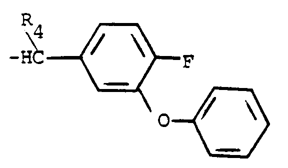 dans laquelle R₄ est défini comme précédemment,
- les radicaux de formule : 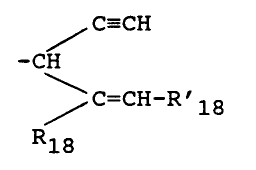 dans laquelle R₁₈ et R'₁₈ sont définis comme précédemment,
- les radicaux de formule : 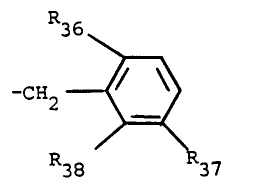 dans laquelle R₃₆ représente un atome d'hydrogène, un atome de fluor ou de chlore, R₃₇ représente un radical phényle ou 3-fluorophényle et R₃₈ représente un atome d'hydrogène, un atome de fluor ou de chlore, ou un radical méthyle.

L'invention a encore notamment pour objet un procédé tel que défini précédemment, caractérisé en ce que l'on utilise au départ un composé de formule R'-OH dans laquelle R' représente un atome d'hydrogène, un radical alkyle linéaire, ramifié ou cyclique renfermant de 1 à 8 atomes de carbone, et notamment un radical éthyle, un radical terbutyle ou encore un radical cyclopropyle ou cyclopropylméthyle, un radical alkyle linéaire ou ramifié renfermant de 1 à 8 atomes de carbone substitué par un ou plusieurs atomes d'halogène, et notamment par un ou plusieurs atomes de fluor, un radical (CH₂)ₘ-O-(CH₂)ₙ-CH₃ dans lequel m représente un nombre entier pouvant varier de 1 à 8 et n représente un nombre entier pouvant varier de 0 à 8, et notamment le radical -CH₂-O-CH₃.

L'invention a tout particulièrement pour objet un procédé tel que défini précédemment, caractérisé en ce que l'on utilise au départ un composé de formule (II) de structure (1R,cis) dans laquelle R représente un reste d'alcool (R,S) ou (S) α-cyano 3-phénoxybenzylique ou un reste d'alcool (R,S) ou (S) α-cyano 4-fluoro 3-phénoxybenzylique et un composé de formule R'-OH dans laquelle R' représente un radical méthyle, éthyle, terbutyle ou 1,1,1,3,3,3-hexafluoropropyle.

Les composés intermédiaires de formule (III) sont des composés nouveaux et l'invention a donc encore pour objet lesdits composés de formule (III), sous forme de mélanges d'isomères ou d'isomères séparés, au niveau du cycle cyclopropane.

Les composés de formule (I) sont pour la plupart connus et décrits dans les brevets européens ou demandes européennes n° 38271, 41021, 48186, 110769, 114012, 215701, 357742, 300898, 176387, 261035, 381563 ou dans le brevet français 2 612 184. Il s'agit de composés biologiquement actifs, possédant, en général, des propriétés pesticides, notamment insecticides et acaricides, ou de composés intermédiaires dans la synthèse de composés actifs.

Les composés de formule (I) dans laquelle R représente un radical méthyle substitué par un atome de brome ou de chlore ou un radical éthyle substitué par 1, 2 ou 3 atomes de brome ou de chlore sont des esters clivables, donc des intermédiaires dans la synthèse d'esters biologiquement actifs. Ils n'ont pas été décrits à ce jour et constituent également l'un des objets de l'invention.

Le clivage des esters de formule (I), intermédiaires dans la synthèse d'esters biologiquement actifs peut être effectué par des méthodes connues de l'homme du métier. Des exemples figurent dans le brevet européen 48186 déjà mentionné plus haut, ou, de manière générale, dans l'ouvrage de T.W. GREENE "Protective Groups in Organic Synthesis".

Les composés de formule (II) utilisés au départ du procédé de l'invention peuvent être obtenus selon un procédé tel que ceux décrits ci-après dans la partie expérimentale, c'est-à-dire par estérification de l'acide correspondant par l'alcool approprié. Ces composés de formule (II) à l'exception de ceux pour lesquels R représente un radical méthyle, éthyle, 3-phénoxybenzyle ou α-cyano 3-phénoxybenzyle sont des composés nouveaux et constituent à ce titre l'un des objets de l'invention.

L'acide mentionné ci-dessus, sous l'une quelconque de ses configurations, est connu ou peut être préparé par un procédé connu de l'homme du métier. L'acide (1R,cis) est décrit dans Agr. Biol. Chem. Vol 29, n° 8, p. 784 (1965) ; l'acide (1S,cis) peut être préparé selon le procédé décrit dans le brevet US 4 296 038 ; l'acide (1R,trans) peut être préparé selon le procédé décrit dans Synth. Comm. (1984) 14, 1239-46 et l'acide (1S,trans) par le même procédé en partant du (-) 3-carène ; l'acide (1R,S cis) peut être préparé selon les procédés décrits dans la référence Agr. Biol. Chem. ci-dessus, ou dans le brevet US 4 296 038, en utilisant au départ le 3-carène racémique, lequel peut être obtenu selon la méthode décrite dans J. Org. Chem. (1987) Vol 52, p. 1493 ou dans Tet. Letters (1984) p. 5255 ; l'acide (1R,S trans), de même que l'acide (1R,S cis) peuvent être obtenus selon le procédé décrit dans Japan Kokaï J81 079644.

Les composés de formule (II) dans laquelle R représente un radical méthyle, 3-phénoxybenzyle et α-cyano 3-phénoxybenzyle sont décrits par exemple dans Synth. Comm. (1988) 18 p. 1139-49 et Tetrahedron (1986) 42 p. 5717-28.

Les exemples suivants illustrent l'invention sans toutefois la limiter.

### EXEMPLE 1 : (1R,cis) 2,2-diméthyl 3-[(Z) 2-(méthoxycarbonyl) éthényl] cyclopropane 1-carboxylate de méthyle.

### Stade A : (1R,cis) 2,2-diméthyl 3-(1,3-dibromo 2-oxopropyl) cyclopropane 1-carboxylate de méthyle.

On mélange sous gaz inerte 2,4 g de (1R,cis) 2,2-diméthyl 3-(2-oxopropyl) cyclopropane 1-carboxylate de méthyle et 30 cm³ de chlorure de méthylène, puis refroidit à +10°C et ajoute 1,3 cm³ de brome. On agite pendant 2 heures à température ambiante puis verse sur un mélange eau-glace. On extrait avec du chlorure de méthylène, sèche la phase organique et évapore à sec. On chromatographie le résidu sur silice en éluant avec du mélange chlorure de méthylène-hexane (7-3) et obtient 3,2 g de produit attendu.

| Analyse pour C₁₀H₁₄Br₂O₃ = 342,04 | | | | | | |
|---|---|---|---|---|---|---|
| Calculé | C% | 35,11 | H% | 4,12 | Br% | 46,72 |
| Trouvé | | 35,0 | | 4,2 | | 46,5 |

Spectre IR (CHCl₃) :
   Absorptions à 1736, 1719 cm⁻¹ (C=O), 1438 cm⁻¹ (COOCH₃)
Spectre RMN (CDCl₃ 250 MHz ppm) :
   1,22 (s), 1,27 (s), 1,34 (s) : CH₃ gem. ; 1,84 (d) et 1,98 (m) : H₁ et H₃ cis ; 3,63 (s), 3,73 (s) : CO₂CH₃ ; 5,50 (d, J=11), 5,67 (d, J=11) : Hal-CH-CH.

### Stade B : (1R,cis) 2,2-diméthyl 3-[(Z) 2-(méthoxycarbonyl) éthényl] cyclopropane 1-carboxylate de méthyle.

On mélange sous gaz inerte 0,16 g de méthylate de sodium et 1,6 cm³ de méthanol. On refroidit à 0°C environ et ajoute lentement 0,5 g de (1R,cis) 2,2-diméthyl 3-(1,3-dibromo 2-oxopropyl) cyclopropane 1-carboxylate de méthyle en solution dans 2,5 cm³ de méthanol. On maintient sous agitation à -5°C environ pendant 1 heure 30 minutes puis chasse le solvant sous pression réduite à 30°C environ et rajoute 10 cm³ de chlorure de méthylène. On lave la solution à l'eau, sèche et la concentre à sec. On obtient 0,324 g de produit brut que l'on chromatographie sur silice en éluant avec du mélange cyclohexane-acétate d'éthyle (6-4). On obtient 0,262 g de produit attendu.
Sepctre IR CHCl₃) :
   Absorptions à 1720 cm⁻¹ (C=O) ; 1634 cm⁻¹ (C=C). Compatible avec l'isomère Z.
Spectre RMN (CDCl₃ 250 MHz ppm) :
   1,28 (s), 1,31 (s) : CH₃ gem. ; 1,96 (d, J=3,5), 3,25 (dd) : H₁/H₃ cis ; 3,46 (s), 3,72 (s) : CH₃ ester en 3 ; 5,90 (dd), 6,65 (dd) : H en 1' - Isomère Z.

### EXEMPLE 2 : (1R,cis) 2,2-diméthyl 3-[(Z) 2-terbutoxycarbonyl) éthényl] cyclopropane 1-carboxylate de méthyle (produit A) et (1R,cis) 2,2-diméthyl 3-[(Z) 2-carboxyéthényl) cyclopropane 1-carboxylate de méthyle (produit B).

On mélange sous gaz inerte 12 cm³ de diméthoxyéthane et 0,684 g de terbutylate de potassium. On y ajoute lentement une solution de 0,684 g de (1R,cis) 2,2-diméthyl 3-(1,3-dibromo 2-oxopropyl) cyclopropane 1-carboxylate de méthyle obtenu au stade A de l'exemple 1 dans 10 cm³ de diméthoxyéthane puis maintient sous agitation pendant 40 minutes à -60°C environ. On ajoute alors 3 cm³ de diméthoxyéthane et 2 cm³ de terbutanol puis maintient sous agitation à -60°C environ pendant 15 minutes. On ajoute de l'eau et du chlorure de méthylène, décante, extrait la phase aqueuse avec du chlorure de méthylène, lave les phases organiques réunies avec de l'eau, sèche et concentre à sec à 30°C environ. On obtient 0,219 g de produit attendu A .

On acidifie, les phases aqueuses réunies, par de l'acide chlorhydrique 2N et extrait avec du chlorure de méthylène. On concentre à sec la phase organique et obtient 0,211 g de produit B correspondant au (1R,cis) 2,2-diméthyl 3-[(Z) 2-carboxyéthényl] cyclopropane carboxylate de méthyle brut. On chromatographie les produits A et B sur silice en éluant respectivement avec les mélanges chlorure de méthylène-hexane (8-2) et cyclohexane-acétate d'éthyle (6-4).

### Analyses du produit A :

Spectre IR (CHCl₃) :
   Absorptions à 1721 cm⁻¹ (épaulement à 1705 cm⁻¹) (C=O), 1631 cm⁻¹ (C=C), 1439 cm⁻¹ (O-CH₃), 1368 cm⁻¹ (O-tBu)
Spectre RMN (CDCl₃ 250 MHz ppm) :
   1,27 (s) et 1,31 (s) : CH₃ gem. ; 1,49 : -CO₂tBu ; 1,92 (d, J=8,5) et 3,26 (m) : H₁ et H₃ cis ; 3,65 : O-CH₃ ; 5,80 (d, J=11,5) : CH= α de CO ; 6,51 (dd, J=10,5-11,5) : CH= β de CO. delta Z.

### Analyses du produit B :

Spectre IR (CHCl₃) :
   Absorptions à 3518 cm⁻¹ (OH acide), 1723-1693 cm⁻¹ (C=O), 1628 cm⁻¹ (C=C), 1440 cm⁻¹ (COOMe)
Spectre RMN (CDCl₃ 250 MHz ppm) :
   1,28 (s) et 1,32 (s) : CH₃ gem. ; 1,99 (d) et 3,21 (m) : H₁ et H₃ cis ; 3,67 (s) : CO₂CH₃ ; 5,92 (d, J=11) et 6,77 (dd) : CO-CH=CH-CH ; 11,6 (m) : 1H mobile.

### EXEMPLE 3 : (1R,cis) 2,2-diméthyl 3-[(Z) 2-[(1,1,1,3,3,3-hexafluoro) propoxycarbonyl] éthényl] cyclopropane 1-carboxy-late de méthyle.

On lave sous gaz inerte 0,057 g d'hydrure de sodium en suspension à 50% dans l'huile par du cyclohexane, puis on ajoute 1 cm³ de diméthoxyéthane. On ajoute à la suspension ainsi obtenue, 0,33 g d'hexafluoro isopropanol dans 1 cm³ de diméthoxyéthane et maintient sous agitation pendant 30 minutes. On refroidit la solution à +5°C et ajoute lentement une solution de 0,173 g de (1R,cis) 2,2-diméthyl 3-(1,3-dibromo 2-oxopropyl) cyclopropane 1-carboxylate de méthyle dans 1 cm³ de diméthoxyéthane. On agite pendant 1 heure à +3/+5°C, verse le mélange réactionnel dans une solution aqueuse d'acide chlorhydrique 1N, extrait au chlorure de méthylène, sèche la phase organique et concentre à sec. On chromatographie le résidu sur silice en éluant avec du mélange chlorure de méthylène-hexane (7-3) et obtient 0,16 g de produit attendu.
Spectre RMN (CDCl₃ 250 MHz) :
   1,30 (s), 1,34 (s) : CH₃ gem. ; 3,68 (s) : CH₃ ester ; 2,06 (d, J=8,5) : H₁ ; 3,12 (dd) : H₃ ; 6,99 (dd) : H'₁ ; 6,00 (d, J=11,5) : H'₂ ; 5,81 : >CH-.

### EXEMPLE 4 : (1R,cis) 2,2-diméthyl 3-[(Z) 2-(1,1,1,3,3,3-hexa-fluoro) propoxycarbonyl] éthényl] cyclopropane 1-carbozylate de méthyle.

On mélange sous gaz inerte 1,5 cm³ de diméthoxyéthane, 0,056 g de terbutylate de potassium et 0,2 g d'hexafluoroisopropanol puis ajoute à 5°C une solution de 0,170 g de (1R,cis) 2,2-diméthyl 3-(1,3-dibromo 2-oxopropyl) cyclopropane 1-carboxylate de méthyle dans 0,5 cm³ de diméthoxyéthane. On maintient 18 heures sous agitation à température ambiante, ajoute de l'acide chlorhydrique N, extrait au chlorure de méthylène, sèche et concentre à sec. On chromatographie le résidu sur silice en éluant au mélange chlorure de méthylène-hexane (7-3) et obtient 0,066 g de produit attendu.
Spectre IR (CHCl₃) :
   Absorptions à 1758, 1740 et 1718 cm⁻¹ (>C=O), 1624 cm⁻¹ (C=C)

### EXEMPLE 5 : (1R,cis) 2,2-diméthyl 3-[(Z) 2-(1,1,1,3,3,3-hexa-fluoro) propoxycarbonyl] éthényl] cyclopropane 1-carboxylate de méthyle.

On opère comme indiqué à l'exemple 4, en utilisant 0,027 g de méthylate de sodium au lieu de terbutylate de potassium. On obtient le produit attendu identique à celui obtenu à l'exemple 4.

### EXEMPLE 6 : (1R,cis) 2,2-diméthyl 3-[(Z) 2-carboxyéthényl] cyclopropane 1-carboxylate de méthyle.

On place sous gaz inerte 1 cm³ de solution 1N de carbonate de potassium, puis ajoute à +5°C environ une solution de 0,342 g de (1R,cis) 2,2-diméthyl 3-(1,3-dibromo 2-oxopropyl) cyclopropane 1-carboxylate de méthyle dans 1,5 cm³ de tétrahydrofuranne. On agite pendant 1 heure 30 minutes puis laisse la température remonter à 20°C et agite pendant 4 heures. On ajoute de l'eau, extrait au chlorure de méthylène, sèche la phase organique et évapore le solvant. On chromatographie le résidu sur silice en éluant au mélange chlorure de méthylène-éthanol (97-3). On obtient 0,046 g de produit attendu.
Spectre RMN (CDCl₃ 250 MHz) :
   1,28 (s), 1,32 (s) : CH₃ gem. ; 1,98 (d, J=8,5), 3,20 (m) : H₁/H₃ cis ; 3,67 (s) : CH₃ ester ; 5,91 (d, J=11,5), 6,77 (dd, J=11,5) : H (C=C).

### EXEMPLE 7 : (1R,cis) 2,2-diméthyl 3-[(Z) 2-(méthoxycarbonyl) éthényl] cyclopropane 1-carboxylate de (S) α-cyano 3-phénoxy-benzyle.

### Stade A : (1R,cis) 2,2-diméthyl 3-(1,3-dibromo 2-oxopropyl) cyclopropane 1-carboxylate de (S) α-cyano 3-phénoxybenzyle.

On mélange sous gaz inerte 1,75 g de 2,2-diméthyl 3-(2-oxopropyl) cyclopropane 1-carboxylate de (S) α-cyano 3-phénoxybenzyle et 17,5 cm³ de tétrahydrofuranne. On ajoute lentement à 0,+5°C, 3,29 g de perbromure d'hydrobromure de pyridinium et maintient à +5°C sous agitation pendant 6 heures. On laisse le mélange en glacière pendant une nuit puis filtre et concentre le filtrat à sec à 25°C sous pression réduite. On purifie le produit sur silice en éluant avec un mélange chlorure de méthylène-hexane (7-3). On obtient 0,8 g de produit attendu.

| Analyse : C₂₃H₂₀NO₄Br₂ = 535,2 | | | | | | | | |
|---|---|---|---|---|---|---|---|---|
| Calculé | C% | 51,6 | H% | 3,8 | Br% | 29,9 | N% | 2,6 |
| Trouvé | | 51,4 | | 4,0 | | 29,4 | | 2,5 |

Spectre RMN (CDCl₃ 250 MHz) :
   Mélange d'isomères au niveau du brome en 1'.
   1,16-1,23-1,25-1,27 : Gem diMe ; 1,88 : H₁ ; 2,07 : H₃ du cyclopropyle ; 6,25 et 6,38 : 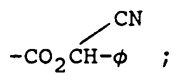 4,06 (d) et 4,39 (d) : -CH₂Br-CO- ; 5,20 (d) et 5,36 (d) : CHBr-CO ; 6,95 à 7,45 : H aromatiques.

L'ester de départ a été obtenu comme suit :

On mélange sous gaz inerte, 15 cm³ de chlorure de méthylène, 2 g d'acide 2,2-diméthyl 3-(2-oxopropyl) cyclopropane 1-carboxylique, 2,59 g de dicyclohexylcarbodiimide et 2,8 cm³ de pyridine, puis ajoute en 20 minutes à +10°C un mélange de 2,74 g d'alcool (S) α-cyano 3-phénoxybenzylique, 20 cm³ de chlorure de méthylène et 0,02 g de diméthylaminopyridine. On agite à 20°C pendant 20 heures puis filtre et concentre le filtrat à sec sous pression réduite à 30°C. On chromatographie le résidu sur silice en éluant avec un mélange cyclohexane-acétate d'éthyle (8-2) et obtient 3,71 g de produit attendu.
Spectre IR (CHCl₃) :
   Absorptions à 1736 cm⁻¹ (carbonyle), 1588 et 1498 cm⁻¹ (phénoxy). Absence d'acide et de OH.
Spectre RMN (CDCl₃ 250 MHz) :
   1,11 (s) et 1,21 (s) : gem diMe ; 1,63 (m) : H₁ et H₃ cis ; 2,16 (s) : CH₃ en α du carbonyle ; 2,87 (m) : CH₂ en α du carbonyle, 6,27 (s) : H de 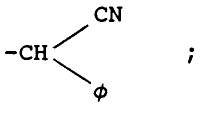 7,00 à 7,45 (9H) : aromatiques.
Dichroïsme circulaire (dioxanne) :
   Max. 225 nm Δε = +1 ; 281 nm Δε = +0,3 ; 288 nm : Δε = +0,3 ; 300 nm Δε = +0,07.

### Stade B : (1R,cis) 2,2-diméthyl 3-[(Z) 2-(méthoxycarbonyl) éthényl] cyclopropane 1-carboxylate de (S) α-cyano 3-phénoxybenzyle.

On mélange sous gaz inerte 0,165 g de méthylate de sodium et 3 cm³ de méthanol puis ajoute à 5°C, 0,770 g de produit obtenu comme décrit au stade A et 6 cm³ de méthanol. On agite 1 heure à 0°C puis 2 heures 30 minutes à température ambiante et verse dans 25 cm³ d'acide chlorhydrique 2N à +5°C environ. On extrait au chlorure de méthylène, lave à l'eau et sèche. On obtient après évaporation du solvant 0,6 g de produit brut que l'on purifie par chromatographie sur silice en éluant au mélange chlorure de méthylène-hexane (8-2) puis (7-3). On obtient 0,267 g d'un mélange renfermant le produit attendu.

### EXEMPLE 8 : (1R,cis) 2,2-diméthyl 3-[(Z) 2-méthoxycarbonyl) éthényl] cyclopropane 1-carboxylate de (R,S) α-cyano 3-phéno- xybenzyle.

### Stade A : (1R,cis 2,2-diméthyl 3-(1,3-dibromo 2-oxopropyl) cyclopropane 1-carboxylate de (R,S) α-cyano 3-phénoxybenzyle.

On mélange sous gaz inerte 0,380 g de (1R,cis) 2,2-diméthyl 3-(2-oxopropyl) cyclopropane 1-carboxylate de (RS) α-cyano 3-phénoxybenzyle et 4 cm³ de tétrachlorure de carbone puis ajoute lentement à 15°C environ, 102 µl de brome dans 1 cm³ de tétrachlorure de carbone et agite pendant 4 heures. On concentre à sec à 30°C environ et obtient 0,530 g de produit attendu brut utilisé tel quel dans le stade suivant. On peut purifier le produit par chromatographie sur silice en éluant avec du mélange chlorure de méthylène-hexane (8-2).
Spectre RMN (CDCl₃ 250 MHz ppm) :
   1,16-1,23-1,25-1,27 : CH₃ gem. ; 1,88-2,07 : H₁ et H₃ cis ; 4,06 (d) et 4,39 (d), 4,22 : -CO-CH₂Br ; 5,20 (d) et 5,36 (d): -CO-CH-Br ; 6,25 et 6,38 : CO₂-CH-CN ; 6,95 à 7,45 : aromatiques.

L'ester de départ a été préparé comme suit :

On mélange sous gaz inerte 0,344 g d'acide 2,2-diméthyl 3-(2-oxopropyl) cyclopropane 1-carboxylique, 3 cm³ de chlorure de méthylène, 0,48 cm³ de pyridine et 0,445 g de dicyclohexylcarbodiimide puis ajoute une solution de 0,471 g d'alcool (RS) α-cyano 3-phénoxybenzylique dans 2 cm³ de chlorure de méthylène et quelques milligrammes de diméthylaminopyridine. On maintient sous agitation à température ambiante pendant 18 heures, filtre et concentre à sec à 30°C. On chromatographie le résidu sur silice en éluant au mélange cyclohexane-acétate d'éthyle (75-25) et obtient 0,594 g de produit attendu.
Spectre RMN (CDCl₃ 250 MHz ppm) :
   1,11 à 1,28 : CH₃ gem. ; 1,50 à 1,68 : H cyclopropyle ; 2,10 (s), 2,16 (s) (3H) : -COCH₃ ; 2,85 (m) : =C-CH₂-CH ; 6,27 (s): -CO₂-CH- ; 7,03 à 7,45 : aromatiques.
Spectre IR (CHCl₃) :
   Absorptions à 1735, 1712 cm⁻¹ (>C=O) ; 1588, 1498 cm⁻¹ (-φ-O-φ).

### Stade B : (1R,cis) 2,2-diméthyl 3-[(Z) 2-méthoxycarbonyl) éthényl] cyclopropane 1-carboxylate de (R,S) α-cyano 3-phénoxybenzyle.

On opère d'une manière analogue à celle décrite au stade B de l'exemple 7, au départ du produit dibromé obtenu au stade A ci-dessus. On obtient après purification dans les mêmes conditions qu'au stade B de l'exemple 7 le produit attendu.

### EXEMPLE 9 : (1R,cis) 2,2-diméthyl 3-[(Z) 2-[(1,1,1,3,3,3-hexafluoro) propoxycarbonyl) éthényl) cyclopropane 1-carboxylate de (RS) α-cyano 3-phénoxybenzyle.

On lave sous gaz inerte 0,030 g d'hydrure de sodium en suspension à 50% dans l'huile par du cyclohexane, puis on y ajoute 1 cm³ de diméthoxyéthane. On ajoute ensuite lentement à 0°C environ une solution de 0,130 g d'hexafluoroisopropanol dans 1 cm³ de diméthoxyéthane et maintient sous agitation pendant 30 minutes à 0,+5°C. On refroidit à -5°C puis ajoute une solution de 0,163 g de (1R,cis) 2,2-diméthyl 3-(1,3-dibromo 2-oxopropyl) cyclopropane 1-carboxylate de (RS) α-cyano 3-phénoxybenzyle obtenu comme décrit au stade A de l'exemple 8, dans 1 cm³ de diméthoxyéthane et maintient sous agitation pendant 1 heure 30 minutes. On verse dans une solution aqueuse 1N d'acide chlorhydrique, extrait au chlorure de méthylène, sèche la phase organique et évapore à sec. On chromatographie le résidu sur silice en éluant au mélange chlorure de méthylène-hexane (8-2) et obtient 0,056 g de produit attendu.
Spectre RMN (CDCl₃) 250 MHz) :
   1,26 (s), 1,30 (s), 1,35 (s), 1,36 (s) : CH₃ gem. ; 2,10 (d), 3,21 (m) : H₁/H₃ cis ; 5,8 (sept) : H de CO₂-CH(CF₃)₂ ; 6,02 (dm), 6,08 (dm) : H en 2' ; 6,32 (s), 6,34 (s) : H en α ; 6,8 à 7,5 (m) : H aromatiques et H en 1'.

### EXEMPLE 10 : (1R,cis) 2,2-diméthyl 3-[(Z) 2-[(1,1,1,3,3,3-hexafluoro propoxy) carbonyl) éthényl] cyclopropane 1-carboxylate de (RS) α-cyano 3-phénoxybenzyle.

### Stade A : (1R,cis) 2,2-diméthyl 3-(1,3-dichloro 2-oxopropyl) cyclopropane 1-carboxylate de (RS) α-cyano 3-phénoxybenzyle.

On mélange sous gaz inerte 0,300 g de (1R,cis) 2,2-diméthyl 3-(2-oxopropyl) cyclopropane 1-carboxylate de (RS) α-cyano 3-phénoxybenzyle obtenu à la préparation de l'exemple 8, 6 cm³ de chloroforme et 160 µl de chlorure de sulfuryle. On agite à température ambiante pendant 1 heure puis concentre à sec. On obtient 0,546 g de produit attendu brut, utilisé tel quel pour le stade suivant.

### Stade B : (1R,cis) 2,2-diméthyl 3-[(Z) 2-[(1,1,1,3,3,3-hexafluoro) propoxycarbonyl] éthényl] cyclopropane 1-carboxylate de (RS) α-cyano 3-phénoxybenzyle.

On mélange sous gaz inerte à -10°C environ 1 cm³ de toluène et 0,02 g d'hydrure de sodium à 50% dans l'huile, puis ajoute lentement 200 µl d'hexafluoro isopropanol et 1 cm³ de toluène. On agite pendant 35 minutes à -10°C puis ajoute 0,1 g de produit brut obtenu au stade A en solution dans 0,5 cm³ de toluène. On laisse revenir à température ambiante et agite pendant 2 heures. On évapore le solvant et chromatographie sur silice en éluant au mélange cyclohexaneacétate d'éthyle (85-15). On obtient le produit attendu identique à celui obtenu à l'exemple 9.

### EXEMPLE 11 : (1R,cis) 2,2-diméthyl 3-[(Z) 2-[(1,1,1,3,3,3-hexafluoro propoxy) carbonyl] éthényl] cyclopropane 1-carbo-xylate de terbutyle.

### Stade A : (1R,cis) 2,2-diméthyl 3-(1,3-dibromo 2-oxopropyl) cyclopropane 1-carboxylate de terbutyle.

On mélange sous gaz inerte 0,113 g de (1R,cis) 2,2-diméthyl 3-(2-oxopropyl) cyclopropane 1-carboxylate de terbutyle, 3 cm³ de tétrahydrofuranne puis ajoute à +5°C 0,34 g de perbromure d'hydrobromure de pyridinium. On agite à température ambiante pendant 1 heure puis essore et concentre à sec. On chromatographie le résidu sur silice en éluant avec un mélange cyclohexane-acétate d'éthyle (7-3) et obtient 0,165 g de produit attendu.
Spectre RMN (CDCl₃ 250 MHz ppm) :
   1,16 à 1,28 : CH₃ gem. ; 1,43 et 1,40 : H de tBu ; 1,7 à 1,9 : H₁ et H₃ ; 4,08 (d), 4,36 (d) et 4,29 : -CO-CH₂-Br ; 5,24 (d) et 5,50 (d) : CO-CHBr-.

Le (1R,cis) 2,2-diméthyl 3-(2-oxopropyl) cyclopropane 1-carboxylate de terbutyle peut être préparé comme suit :

On mélange sous gaz inerte 0,17 g d'acide (1R,cis) 2,2-diméthyl 3-(1-oxopropyl) cyclopropane 1-carboxylique et 1,7 cm³ d'acétate d'éthyle puis ajoute lentement une solution de 0,36 g de terbutyloxy N,N'-diisopropylcarbodiimide dans 1 cm³ d'acétate d'éthyle. Après 6 heures sous agitation à température ambiante, on rajoute 0,15 g de terbutyloxy N,N'-diisopropylcarbodiimide et poursuit l'agitation 16 heures. On essore puis évapore le solvant. On chromatographie le résidu sur silice en éluant avec un mélange cyclohexane-acétate d'éthyle (7-3) et obtient 0,153 g de produit attendu.
Spectre IR (CHCl₃) :
   Absorption à 1712-1368 cm⁻¹ (CH₃ de tBu) >=O - Absence d'acide.
Spectre RMN (CDCl₃ 250 MHz ppm) :
   1,18 (s) et 1,20 (s) : CH₃ gem. ; 1,35 (m), 1,50 (d, J=8,5) : H₃ et H₁ ; 1,45 (s) : H de tBu ; 2,15 (s) : H de CH₃ en 3' ; 2,79 (dd) et 2,93 (dd) : H de CH₂ en 1'.

Le terbutyloxy N,N'-diisopropylcarbodiimide peut être préparé comme suit :

On mélange sous gaz inerte 20 g de terbutanol et 1,73 g de chlorure cuivreux puis ajoute à +30°/+35°C, 34 g de N,N'-diisopropylcarbodiimide et maintient sous agitation en laissant revenir à température ambiante. On essore, rince le filtre à l'éther isopropylique, évapore le solvant et rectifie le résidu sous 4-5 mm de Hg. Eb = 47-48°C. On obtient 41,08 g de produit attendu.
Spectre RMN (CDCl₃ 250 MHz) :
   1,05 (d), 1,09 (d) : CH₃ ; 3,14 (m), 3,68 (m) : -CH< ; 3,24 (d) : NH ; 1,47 (s) : tBu.
Spectre IR (CHCl₃) :
   Absorptions à 3436 cm⁻¹ (NH) ; 1656 cm⁻¹ (C=N).

### Stade B : (1R,cis) 2,2-diméthyl 3-[(Z) 2-[(1,1,1,3,3,3-hexafluoro propoxy) carbonyl] éthényl] cyclopropane 1-carboxylate de terbutyle.

On mélange sous gaz inerte 0,055 g d'hydrure de sodium (en suspension à 50% dans l'huile) et 1 cm³ de toluène puis ajoute à +5,+10°C une solution de 0,162 g d'hexafluoro isopropanol dans 2 cm³ de toluène et maintient sous agitation à +10°C pendant 30 minutes. On ajoute une solution de 0,184 g de (1R,cis) 2,2-diméthyl 3-(1,3-dibromo 2-oxopropyl) cyclopropane 1-carboxylate de terbutyle dans 1,5 cm³ de toluène et agite pendant 2 heures à température ambiante. On verse le mélange sur de l'acide chlorhydrique 2N à +10°C, extrait à l'acétate d'éthyle, lave la phase organique à l'eau, la sèche et l'évapore à sec. On chromatographie le résidu sur silice en éluant avec un mélange cyclohexane-acétate d'éthyle (95-5) et obtient 0,135 g de produit attendu. F = 95°C.
Spectre IR (CHCl₃) :
   Absorption à 1742, 1710 cm⁻¹ (C=O) et 1622 cm⁻¹ (C=C).
Spectre RMN (CDCl₃ 250 MHz) :
   1,28 (s), 1,32 (s) : CH₃ gem. ; 1,45 (s) : H de tBu ; 1,97-3,03 : H₁/H₃ cis ; 5,81 (sept) : H de -CH(CF₃)₂ ; 5,99 (dd, J=11) : H en 2' ; 7,00 (dd, J=11 et 10,5) : H en 1', isomère Z.

### EXEMPLE 12 : (1R,cis) 2,2-diméthyl 3-[(Z) 2-[(1,1,1,3,3,3- hexafluoro propoxy) carbonyl] éthényl] cyclopropane 1-carbo- xylate de 2,2,2-trichloroéthyle.

### Stade A : (1R,cis) 2,2-diméthyl 3-(1,3-dibromo 2-oxopropyl) cyclopropane 1-carboxylate de 2,2,2-trichloroéthyle.

On mélange sous gaz inerte 0,2 g de (1R,cis) 2,2-diméthyl 3-(2-oxopropyl) cyclopropane 1-carboxylate de 2,2,2-trichloroéthyle, 3 cm³ de chlorure de méthylène et 1 cm³ de chloroforme, puis ajoute à +5°C environ, 70 µl de brome et agite à température ambiante pendant 3 heures. On ajoute un mélange eau-glace, décante et extrait avec du chlorure de méthylène. On sèche la phase organique et évapore le solvant. On chromatographie le résidu sur silice en éluant avec un mélange chlorure de méthylène-cyclohexane (7-3) et obtient 0,23 g de produit attendu.
Spectre RMN (CDCl₃ 250 MHz ppm) :
   1,22 (s), 1,31 (s) et 1,33 (s) : CH₃ gem. ; 1,94 à 2,10 (m) : H₁ et H₃ ; 4,06 (d), 4,39 (d), 4,74 (d), 4,87 (d), 4,26 (s), 4,70 (s) : 2 CH₂-X ; 5,24 (d) et 5,47 (d) :

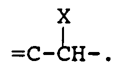

L'ester de départ est obtenu comme suit :

On mélange sous gaz inerte 0,2 g d'acide (1R,cis) 2,2-diméthyl 3-(2-oxopropyl) cyclopropane 1-carboxylique, 4 cm³ de chlorure de méthylène, 280 µl de pyridine et 0,267 g de dicyclohexylcarbodiimide. Après 5 minutes, on ajoute 135 µl de trichloroéthanol et quelques cristaux de diméthylaminopyridine. On agite pendant 20 heures à température ambiante, essore et évapore à sec. On chromatographie le résidu sur silice en éluant avec un mélange cyclohexane-acétate d'éthyle (8-2) et obtient 0,316 g de produit attendu.
Spectre IR (CHCl₃) :
   Absence d'acide et d'OH. Absorption à 1732 et 1719 cm⁻¹ (C=O).
Spectre RMN (CDCl₃ 250 MHz ppm) :
   1,18 (s), 1,26 (s) : CH₃ gem. ; 1,58 (m), 1,75 (d) : H₃ et H₁ cis ; 2,15 (s) : CH₃-C=O ; 2,89 (m) : =C-CH₂-C ; 4,70 (s) : -CO₂-CH₂-.

### Stade B : (1R,cis) 2,2-diméthyl 3-[(Z) 2-[(1,1,1,3,3,3-hexa-fluoro propoxy) carbonyl] éthényl] cyclopropane 1-carboxylate de 2,2,2-trichloroéthyle.

On mélange sous gaz inerte 0,036 g d'hydrure de sodium à 50% et 1 cm³ de toluène, puis ajoute à 10°C environ une solu-tion de 0,135 g d'hexafluoro isopropanol dans 0,5 cm³ de toluène et agite pendant 30 minutes à la même température. On ajoute ensuite une solution de 0,170 g de produit obtenu au stade A dans 1 cm³ de toluène, agite pendant 1 heure puis laisse à température ambiante et agite de nouveau pendant 1 heure. On ajoute ensuite à +10°C, de l'acide chlorhydrique 0,1N jusqu'à obtention de pH1 et agite 15 minutes. On extrait à l'acétate d'éthyle, sèche la phase organique et concentre à sec. On chromatographie le résidu sur silice en éluant avec un mélange chlorure de méthylène-cyclohexane (7-3) et obtient 0,1 g de produit attendu.
Sepctre IR (CHCl₃) :
   Absorption à 1746 cm⁻¹ (C=O) et 1627 cm⁻¹ (C=C).
Spectre RMN (CDCl₃ 250 MHz ppm) :
   1,35 (s) : CH₃ gem. ; 2,19 (d) et 3,23 (m) : H₃ et H₁ cis ; 4,74 (A-B) : -CO₂-CH₂-C ; 5,8 'sept.) : F₃C-CH-CF₃ ; 6,04 (d, J=11,5) : H en 2' ; 6,93 (dd) : H en 1'.

### EXEMPLE 13 : (1R,cis) 2,2-diméthyl 3-[(Z) 2-(méthoxycarbonyl) éthényl] cyclopropane 1-carboxylate de 2,2,2-trichloroéthyle.

On mélange sous gaz inerte 10 cm³ de méthanol et 0,724 g de méthylate de sodium. On ajoute lentement à 0,+5°C, 4 g de produit obtenu comme décrit au stade A de l'exemple 12, dans 30 cm³ de méthanol et maintient sous agitation pendant 16 heures à +5°C environ. On verse dans un mélange d'eau et de glace additionné de 15 cm³ d'acide chlorhydrique 2N. On maintient sous agitation pendant 15 minutes puis extrait au chlorure de méthylène, lave la phase organique à l'eau, la sèche et l'amène à sec. On chromatographie le résidu sur silice en éluant au mélange chlorure de méthylène-cyclohexane (1-1) et obtient 0,879 g de produit attendu.
Spectre RMN (CDCl₃ 250 MHz ppm) :
   1,33 (s) : CH₃ gem. ; 2,10 (d) : H₁ ; 3,36 (m) : H₃ ; 3,73 (s) : CH₃-O- ; 4,73 (A-B) : -CO₂-CH₂-C ; 5,93 (d, J=11,5) : H en 2' ; 6,59 (dd) : H en 1'. Structure cis, delta Z.

### EXEMPLE 14 : (1R,cis) 2,2-diméthyl 3-[(Z) 2-[(1,1,1,3,3,3- hexafluoro propoxy) carbonyl] éthényl] cyclopropane 1-carboxylate de 2-chloroéthyle.

### Stade A : (1R,cis) 2,2-diméthyl 3-(1,3-dibromo 2-oxopropyl) cyclopropane 1-carboxylate de 2-chloroéthyle.

On mélange sous gaz inerte 0,235 g de (1R,cis) 2,2-diméthyl 3-(2-oxopropyl) cyclopropane 1-carboxylate de 2-chloroéthyle et 5 cm³ de chloroforme puis ajoute lentement 100 µl de brome. On agite à température ambiante pendant 2 heures puis ajoute un mélange eau-glace, agite pendant 15 minutes, décante et extrait au chlorure de méthylène. On sèche la phase organique et évapore le solvant. On chromatographie le résidu sur silice en éluant avec un mélange chlorure de méthylène-hexane (7-3) et obtient 0,067 g de produit attendu.
Spectre RMN (CDCl₃ 250 MHz ppm) :
   1,20 (s), 1,26 (s), 1,29 (s), 1,30 (s) : CH₃ gem. ; 1,84 à 2,00 (m) : H₁ et H₃ ; 3,64 (t), 3,72 (t) ; -CH₂Cl ; 4,28 (t), 4,40 (m) : -CO₂-CH₂- ; 4,07 (d), 4,20 à 4,45 (m), 4,28 (m) : C-CH₂-X ; 5,23 (d) et 5,47 (d) : =C-CHX-.

L'ester de départ est obtenu comme suit :

On mélange sous gaz inerte 0,171 g d'acide (1R,cis) 2,2-diméthyl 3-(2-oxopropyl) cyclopropane 1-carboxylique et 0,7 cm³ de 1-bromo 2-chloroéthane puis ajoute lentement à 0,+5°C, 145 µl de triéthylamine et agite à température ambiante. Après quelques heures, on rajoute 20 µl de triéthylamine et 0,2 cm³ de bromochloroéthane. On agite en tout 20 heures à température ambiante, puis ajoute de l'eau, décante et extrait au chlorure de méthylène. On sèche la phase organique et évapore à sec. On chromatographie le résidu sur silice en éluant au mélange cyclohexane-acétate d'éthyle (75-25) et obtient 0,172 g de produit attendu.
Spectre RMN (CDCl₃ 250 MHz ppm) :
   1,16 (s), 1,23 (s) : CH₃ gem. ; 1,63 (d, J=8,5), 1,48 (m) : H₁ et H₃ cis ; 2,15 (s) -CH₃-CO- ; 2,88 (m) : -CH₂-CO- ; 3,66 (t): -CH₂-Cl ; 4,28 (m) : CO₂-CH₂-.
Spectre IR (CHCl₃) :
   Absorption à 1720 cm⁻¹ (C=O), absence d'acide.

### Stade B : (1R,cis) 2,2-diméthyl 3-[(Z) 2-[(1,1,1,3,3,3-hexafluoro propoxy) carbonyl] éthényl] cyclopropane 1-carboxylate de 2-chloroéthyle.

On mélange sous gaz inerte 3 cm³ de toluène et 0,11 g d'hydrure de sodium à 50%, puis ajoute lentement à +5,+7°C, une solution de 0,5 g de 1,1,1,3,3,3-hexafluoro isopropanol dans 1 cm³ de toluène. On agite à la même température pendant 45 minutes, puis ajoute lentement une solution de 0,341 g de produit obtenu comme décrit au stade A, dans 2 cm³ de toluène et agite pendant 2 heures à +5,+7°C et 1 heure à température ambiante. On refroidit à +10°C, ajoute un peu d'acide acétique dilué au demi, décante et exrait à l'acétate d'éthyle. On sèche la phase organique et concentre à sec. On chromatographie le résidu sur silice en éluant avec un mélange chlorure de méthylène-cyclohexane (1-1) et obtient 0,11 g de produit attendu.
Spectre RMN (CDCl₃ 250 MHz ppm) :
   1,32 (s), 1,34 (s) : CH₃ gem. ; 2,10 (d), 3,15 (m) : H₁ et H₃ cis ; 3,69 (t): -CH₂-Cl ; 4,33 (t) : CO₂-CH₂- ; 5,81 (m) : -CO₂-CH< ; 6,01 (d, J=11) : H en 2' ; 6,95 (dd) : H en 1'.
Spectre IR (CHCl₃) :
   Absorption à 1760, 1744 et 1726 cm⁻¹ (C=O complexe), 1625 cm-¹ (C=C).

### EXEMPLE 15 : (1R,cis) 2,2-diméthyl 3-[(Z) 2-[(1,1,1,3,3,3-hexafluoropropoxy) carbonyl) éthényl] cyclopropane 1-carboxylate de (S) α-cyano 3-phénoxybenzyle.

On opère de manière analogue à celle décrite à l'exemple 9, en utilisant au départ l'ester obtenu au stade A de l'exemple 7. On obtient après purification dans les mêmes conditions qu'à l'exemple 9 le produit attendu.

### EXEMPLE 16 : (1R,cis) 2,2-diméthyl 3-[(Z) 2-[(1,1,1,3,3,3-hexafluoro propoxy) carbonyl] éthényl] cyclopropane carboxy-late de 3,4,5,6-tétrahydrophtalimido méthyle.

### Stade A : (1R,cis) 2,2-diméthyl 3-(1,3-dibromo 2-oxopropyl) cyclopropane 1-carboxylate de 3,4,5,6-tétrahydrophtalimido méthyle.

On mélange sous gaz inerte 0,8 g de (1R,cis) 2,2-diméthyl 3-(2-oxopropyl) cyclopropane 1-carboxylate de 3,4,5,6-tétrahydrophtalimido méthyle et 8 cm³ de tétrahydrofuranne puis ajoute lentement 1,72 g de perbromure de pyridinium, sous forme d'hydrobromure. Après 6 heures sous agitation à +5°C, on essore le précipité et le lave au tétrahydrofuranne, puis concentre le filtrat à sec à 25°C sous pression réduite. On chromatographie le résidu sur silice en éluant au chlorure de méthylène et obtient 0,31 g de produit attendu.
Spectre RMN (CDCl₃ 250 MHz ppm) :
   1,22 (s), 1,19 (s), 1,25 (s) et 1,30 (s) : CH₃ gem. ; 1,76 (d) et 1,34 (m) : H₁ et H₃ ; 1,78 (m) : CH₂ en β et 2,38 (m) CH₂ en α dans le cycle tétrahydrophtalimide ; 4,07 (d), 4,36 (d) et 4,28 (système AB) : -COCH₂Br ; 5,22 (d), 5,55 (d) et 5,57 (système AB) : -CO₂CH₂- ; 5,40 (d) et 5,42 (d) -COCHBr-.

L'ester de départ a été obtenu comme suit :

On mélange sous gaz inerte 1 g d'acide 2,2-diméthyl 3-(2-oxopropyl) cyclopropane 1-carboxylique, 8 cm³ de chlorure de méthylène, 1,395 cm³ de pyridine et 1,29 g de dicyclohexylcarbodiimide, puis introduit à +5°C une solution de 1,1 g d'alcool 3,4,5,6-tétrahydrophtalimido méthylique dans 5 cm³ de chlorure de méthylène. On ajoute quelques milligrammes de diméthylaminopyridine puis agite pendant 18 heures en laissant remonter la température. On filtre et concentre le filtrat à sec sous pression réduite à 30°C. On chromatographie le résidu sur silice en éluant au mélange cyclohexaneacétate d'éthyle (7-3) et obtient 1,336 g de produit attendu.
Spectre RMN (CDCl₃ 250 MHz ppm) :
   1,22 (s), 1,19 (s), 1,25 (s) et 1,30 (s) : CH₃ gem. ; 1,76 (d) et 1,34 (m) : H₁ et H₃ ; 1,78 (m) et 2,38 (m) : CH₂ en β et CH₂ en α dans le cycle tétrahydrophtalimide ; 4,07 (d), 4,36 (d) et 4,28 (système AB) : -COCH₂Br ; 5,22 (d), 5,55 (d) et 5,57 (système AB) : -CO₂CH₂- ; 5,40 (d) et 5,42 (d) : -COCHBr-.

### Stade B : (1R,cis) 2,2-diméthyl 3-[(Z) 2-[(1,1,1,3,3,3-hexafluoro propoxy) carbonyl] éthényl] cyclopropane carboxylate de 3,4,5,6-tétrahydrophtalimido méthyle.

On mélange sous gaz inerte 1 cm³ de toluène et 20 mg d'hydrure de sodium à 50% dans l'huile. On refroidit à +5,+10°C et ajoute lentement une solution de 67 mg d'hexafluoro isopropanol dans 0,5 cm³ de toluène. On agite pendant 30 minutes à 10°C puis ajoute une solution de 0,1 g de produit obtenu au stade A, dans 1 cm³ de toluène. On agite en laissant remonter la température. Après 2 heures, on verse dans un mélange acide chlorhydrique 2N-glace, agite pendant 5 minutes et extrait à l'acétate d'éthyle. On lave la phase organique à l'eau, la sèche et la concentre à sec à 30°C sous pression réduite. On chromatographie le résidu sur silice en éluant avec un chlorure de méthylène. On obtient 0,04 g de produit attendu.
Spectre RMN (CDCl₃ 250 MHz ppm) :
   1,28 (s) et 1,32 (s) : CH₃ gem. ; 1,98 (d, J=8,5) et 3,13 (m): H₁ et H₃ (cis) ; 1,78 (m) et 2,38 (m) : CH₂ en β et CH₂ en α dans le cycle tétrahydrophtalimide ; 5,52 (système AB) : -CO₂-CH₂-N ; 6,01 (d, J=11,5) : H en 1' ; 6,94 (dd, J=10,5 et 11,5) : H en 2' ; 5,80 (m) : -CO₂-CH-(CF₃)₂.

### EXEMPLE 17 : (1R,cis) 2,2-diméthyl 3-[(Z) 2-(méthoxycarbonyl) éthényl] cyclopropane 1-carboxylate de pentafluorobenzyle.

### Stade A : (1R,cis) 2,2-diméthyl 3-(1,3-dibromo 2-oxopropyl) cyclopropane 1-carboxylate de pentafluorobenzyle.

On mélange sous gaz inerte 1,2 g de (1R,cis) 2,2-diméthyl 3-(2-oxopropyl) cyclopropane 1-carboxylate de pentafluorobenzyle et 12 cm³ de tétrahydrofuranne. On ajoute lentement à +5°C, 2,45 g de perbromure d'hydrobromure de pyridinium, puis maintient sous agitation à +5°C pendant 7 heures. On filtre et concentre le filtrat à sec sous pression réduite à 25°C. On chromatographie le résidu sur silice en éluant avec un mélange chlorure de méthylène-hexane (7-3) et obtient 1,08 g de produit attendu.
Spectre RMN (CDCl₃ 250 MHz ppm) :
   1,20 (s), 1,24 (s), 1,26 (s) et 1,30 (s) : CH₃ gem. ; 1,73 (d) et 1,96 (m) : H₁ et H₃ ; 4,07 (d), 4,37 (d) et 4,26 (s) : CO₂-CH₂ ; 5,15 et 5,22 : -COCH₂Br- ; 5,20 (d) et 5,41 (d) : -COCHBr-.

L'ester de départ a été obtenu comme suit :

On mélange sous gaz inerte à 0,+5°C, 0,165 g d'acide (1R,cis) 2,2-diméthyl 3-(2-oxopropyl) cyclopropane 1-carboxylique, 2 cm³ de chlorure de méthylène, 230 µl de pyridine et 0,213 g de dicyclohexylcarbodiimide puis ajoute un mélange constitué de 0,2 g d'alcool pentafluorobenzylique, 1 cm³ de chlorure de méthylène et quelques milligrammes de diméthylaminopyridine. On laisse remonter la température et agite pendant 12 heures. On filtre, rince le filtre au chlorure de méthylène et concentre à sec le filtrat. On purifie le résidu par chromatographie sur silice en éluant avec un mélange cyclohexane-acétate d'éthyle (80-20) et obtient 0,25 g de produit attendu.
Spectre RMN (CDCl₃ 250 MHz ppm) :
   1,16 (s) et 1,20 (s) : CH₃ gem. ; 1,48 (m) et 1,68 (d) : H du cyclopropyle (cis) ; 2,14 (s) : -CO-CH₃ ; 2,88 (m) : -COCH₂-; 5,15 (système AB) : -CO₂CH₂-.

### Stade B : (1R,cis) 2,2-diméthyl 3-[(Z) 2-(méthoxycarbonyl) éthényl] cyclopropane 1-carboxylate de pentafluorobenzyle.

On mélange sous gaz inerte 0,022 g de méthylate de sodium et 0,3 cm³ de méthanol puis ajoute lentement à 0,+5°C, une solution de 0,1 g de produit obtenu au stade A dans 1cm³ de méthanol. On agite pendant 2 heures puis verse dans un mélange d'eau, de glace et de 0,75 cm³ d'acide chlorhydrique 2N. On agite pendant 15 minutes et extrait au chlorure de méthylène. On lave la phase organique à l'eau et la sèche, puis la concentre à sec à 30°C sous pression réduite. On chromatographie le résidu sur silice en éluant avec un mélange chlorure de méthylène-hexane(7-3) et obtient 0,031 g de produit attendu.
Spectre RMN (CDCl₃ 250 MHz ppm) :
   1,28 (s) et 1,31 (s) : CH₃ gem ; 1,93 (d, J=8,5) et 3,28 (m) : H₁ et H₃ cis ; 3,72 (s) : -COOCH₃ ; 5,18 (s) : -CO₂-CH₂- ; 5,91 (d, J=12) : H en 1' ; 6,60 (dd, J=12 et 10) : H en 2'.

### EXEMPLE 18 : (1R,cis) 2,2-diméthyl 3-[(Z) 2-[(1,1,1,3,3,3-hexafluoro) propoxycarbonyl) éthényl] cyclopropane 1-carboxylate de pentafluorobenzyle.

On mélange sous gaz inerte 0,03 g d'hydrure de sodium à 50% dans l'huile et 1 cm³ de toluène. On ajoute lentement, à +5,+10°C, une solution de 0,099 g d'hexafluoro isopropanol dans 1 cm³ de toluène, agite pendant 30 minutes puis ajoute une solution de 0,15 g de produit obtenu au stade A de l'exemple 17 dans 1 cm³ de toluène. On agite pendant 2 heures en laissant remonter la température puis verse dans un mélange d'acide chlorhydrique 2N et de glace. On extrait à l'acétate d'éthyle, lave la phase organique à l'eau et la sèche puis la concentre à sec sous pression réduite. On chromatographie le résidu sur silice et obtient 0,102 g de produit attendu.
Spectre RMN (CDCl₃ 250 MHz ppm) :
   1,30 (s) et 1,33 (s) : CH₃ gem ; 2,03 (d, J=8,5) et 3,15 (m): H₁ et H₃ cis ; 5,20 (système AB) : -CO₂-CH₂- ; 5,80 -CO₂-CH ; 6,03 (d, J=11,5) : H en 1' ; 6,03 (m) : H en 2' delta Z.

### EXEMPLE 19 : (1R,cis) 2,2-diméthyl 3-[Z) 2-(méthoxycarbonyl) éthényl] cyclopropane 1-carboxylate de terbutyle.

On mélange sous gaz inerte 0,02 g de sodium et 0,5 cm³ de toluène, puis ajoute lentement, à 5°C environ, 0,2 cm³ de méthanol. On agite pendant 30 minutes à température ambiante puis refroidit à 5°C et ajoute une solution de 0,0768 g de produit obtenu au stade A de l'exemple 11 dans 1 cm³ de toluène. On agite pendant 3 heures en laissant remonter la température, puis acidifie à pH 3-4 par addition d'acide chlorhydrique 2N. On ajoute 5 cm³ de toluène, décante, lave la phase organique à l'eau, sèche et évapore le solvant. On chromatographie le résidu sur silice en éluant avec un mélange cyclohexane-éther isopropylique (8-2) et obtient 0,03 g de produit attendu.
Spectre RMN (CDCl₃ 250 MHz ppm) :
   1,26 (s), 1,29 (s) : CH₃ gem. ; 1,80 (d, J=8,5), 3,14 (dd) : H₁ et H₃ cis ; 1,44 (s) : CO₂tBu ; 3,71 (s) : CO₂CH₃ ; 5,89 (d, J=11,5), 6,65 (dd, J=11,5 et 10,5) : H en α et β de CO₂CH₃, structure ΔZ.

## Revendications

1. Procédé de préparation des composés de formule (I) : 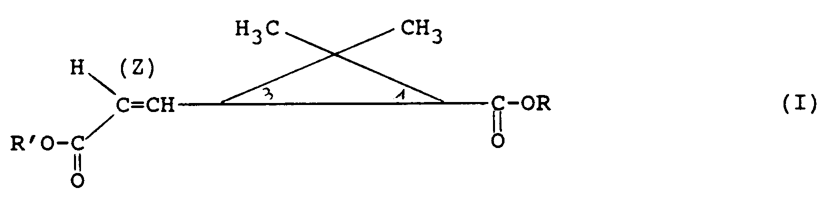 dans laquelle R représente ou un reste d'ester clivable ou un reste d'ester connu dans la chimie des pyréthrinoïdes, à savoir :
a) soit un radical benzyle éventuellement substitué sur les sommets aromatiques par un ou plusieurs radicaux choisis dans le groupe constitué par les radicaux alkyles comportant de 1 à 4 atomes de carbone, les radicaux alcényles comportant de 2 à 6 atomes de carbone, les radicaux alcényloxy comportant de 2 à 6 atomes de carbone, les radixaux alcadiényles comportant de 4 à 8 atomes de carbone, le radical méthylènedioxy et les atomes d'halogène,
b) soit un groupement 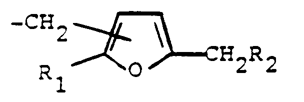 dans lequel le substituant R₁ représente un atome d'hydrogène ou un radical méthyle et le substituant R₂ un aryle monocyclique ou un groupement -C≡CH,
c) soit un groupement 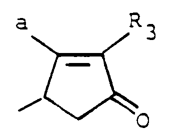 dans lequel a représente un atome d'hydrogène ou un radical méthyle et R₃ représente un radical organique aliphatique comportant de 2 à 6 atomes de carbone et une ou plusieurs insaturations carbone-carbone,
d) soit un groupement 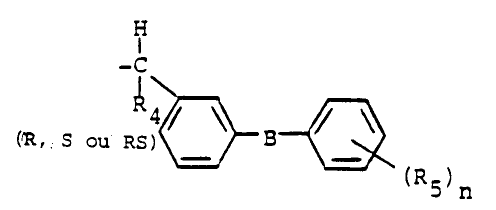 dans lequel B représente un atome d'oxygène ou de soufre ou un groupement 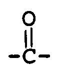 ou -CH₂-, R₄ représente un atome d'hydrogène, un atome de chlore, de brome ou d'iode, un radical -C≡N, un radical méthyle, un radical -CONH₂, un radical -CSNH₂ ou un radical -C≡CH, R₅ représente un atome d'halogène ou un radical méthyle et n représente un nombre égal à 0, 1 ou 2,
e) soit un groupement 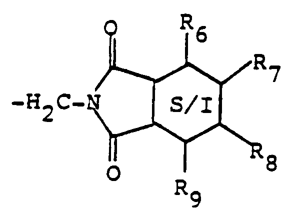 dans lequel les substituants R₆, R₇, R₈ et R₉ représentent un atome d'hydrogène, un atome de chlore ou un radical méthyle et dans lequel S/I symbolise un cycle aromatique ou un cycle analogue dihydro, tétrahydro ou hexahydro,
f) soit un groupement (succimido ou maléimido) méthylène,
g) soit un groupement 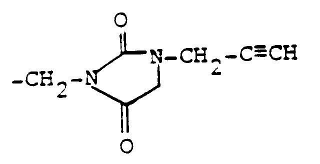
h) soit un groupement 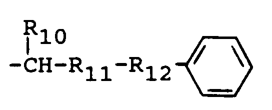 dans lequel R₁₀ représente un atome d'hydrogène ou un radical CN, R₁₂ représente un radical -CH₂- ou un atome d'oxygène, R₁₁ représente un radical thiazolyle ou thiadiazolyle dont la liaison avec 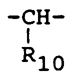 peut se trouver à l'une quelconque des positions disponibles, R₁₂ étant lié à R₁₁ par l'atome de carbone compris entre l'atome de soufre et un atome d'azote,
i) soit un groupement 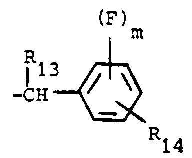 dans lequel R₁₃ représente un atome d'hydrogène ou un radical -CN ou -C≡CH, R₁₄ représente un radical trifluorométhyle ou un radical alkyle, alcényle ou alcynyle renfermant au maximum 6 atomes de carbone et m représente un nombre égal à 1, 2, 3 ou 4,
j) soit un groupement 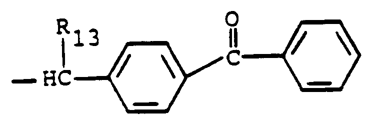 dans lequel R₁₃ est défini comme ci-dessus,
k) soit un groupement 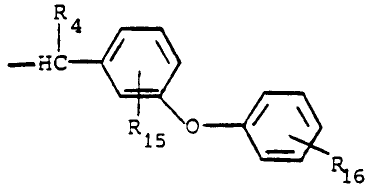 dans lequel R₄ est défini comme ci-dessus, R₁₅ représente un atome de fluor, de chlore ou de brome et R₁₆ représente un atome d'hydrogène, de fluor, de chlore ou de brome,
l) soit un groupement 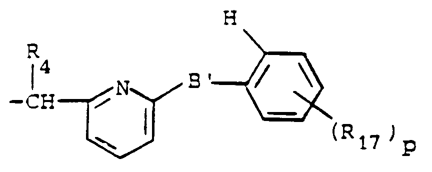 dans lequel R₄ est défini comme ci-dessus, chacun des R₁₇ représente indépendamment un groupement alkyle renfermant de 1 à 4 atomes de carbone, alcoxy renfermant de 1 à 4 atomes de carbone, alkylthio renfermant de 1 à 4 atomes de carbone, alkylsulfonyl renfermant de 1 à 4 atomes de carbone, trifluorométhyle, 3,4-méthylènedioxy, chloro, fluoro ou bromo, p représente un nombre égal à 0, 1 ou 2 et B' représente un atome d'oxygène ou un atome de soufre,
m) soit un groupement 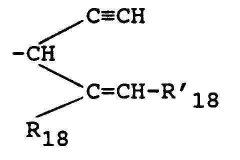 dans lequel R₁₈ représente un atome de fluor ou un radical méthyle et R'₁₈ représente un radical méthyle, éthyle ou propargyle,
n) soit un groupement 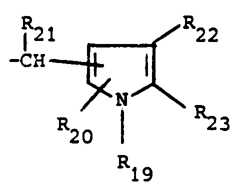 dans laquelle R₂₁ représente un atome d'hydrogène, un groupement -C≡N, -C≡CH, -CF₃ ou un radical alkyle renfermant de 1 à 3 atomes de carbone, R₂₀, R₂₂ et R₂₃ identiques ou différents les uns des autres, représentent un atome d'hydrogène, un atome d'halogène, un radical alkyle renfermant jusqu'à 18 atomes de carbone, un radical aryle renfermant jusqu'à 14 atomes de carbone, un radical aralkyle renfermant jusqu'à 18 atomes de carbone, un radical cyano, un radical -CF₃, un radical -CO₂-alkyle renfermant jusqu'à 8 atomes de carbone, un radical NO₂, un radical alcoxy renfermant jusqu'à 8 atomes de carbone, un radical 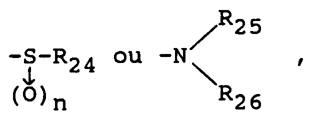 n étant égal à 0, 1 ou 2 et les radicaux R₂₄, R₂₅ et R₂₆ représentant un radical alkyle renfermant de 1 à 8 atomes de carbone, les radicaux R₂₂ et R₂₃ pouvant former un homocycle carboné saturé ou insaturé renfermant jusqu'à 8 atomes de carbone et R₁₉ représente
- ou bien un radical 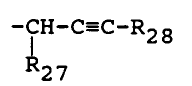 dans lequel R₂₇ et R₂₈, identiques ou différents l'un de l'autre, représentent un atome d'hydrogène, un atome d'halogène, un radical alkyle renfermant de 1 à 8 atomes de carbone ou un radical aryle renfermant jusqu'à 14 atomes de carbone
- ou bien un radical 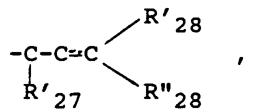 dans lequel R'₂₇, R'₂₈ et R''₂₈, identiques ou différents les uns des autres, représentent l'une des valeurs indiquées ci-dessus pour R₂₇ et R₂₈, les traits pointillés représentent une seconde liaison éventuelle,
- ou bien un radical 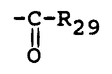 dans lequel R₂₉ peut avoir les valeurs indiquées précédemment pour R₂₂ et R₂₃, à l'exception d'halogène, cyano, NO₂, 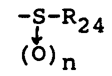 dans lequel n est égal à 1 ou 2 et 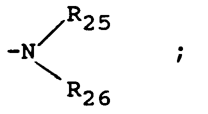
- ou bien un radical 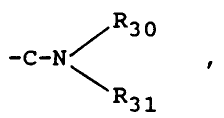 dans lequel R₃₀ et R₃₁, identiques ou différents l'un de l'autre, représentent un atome d'hydrogène, un radical alkyle renfermant de 1 à 18 atomes de carbone, un radical aryle renfermant jusqu'à 14 atomes de carbone, un radical aralkyle renfermant jusqu'à 18 atomes de carbone, un radical CF₃, un radical CO₂-alkyle renfermant jusqu'à 8 atomes de carbone ou un radical alcoxy renfermant jusqu'à 8 atomes de carbone,
o) soit un groupement 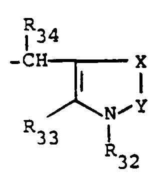 dans laquelle X représente un atome de soufre ou d'oxygène, Y représente un groupe >C=O, >C=S ou >CH₂, R₃₂ représente un atome d'hydrogène, un radical alkyle, linéaire, ramifié ou cyclique, saturé ou insaturé, renfermant jusqu'à 8 atomes de carbone, éventuellement substitué par un ou plusieurs halogènes, ou un radical aryle renfermant jusqu'à 14 atomes de carbone, R₃₃ représente un atome d'hydrogène, un radical alkyle linéaire, ramifié ou cyclique, saturé ou insaturé, renfermant jusqu'à 8 atomes de carbone, éventuellement substitué par un ou plusieurs halogènes, ou R₃₃ représente un radical aryle renfermant jusqu'à 14 atomes de carbone, un radical -CF₃, -NO₂, -C≡N, un atome d'halogène, un radical alcoxyle renfermant jusqu'à 8 atomes de carbone ou un radical -CO₂-alkyle renfermant jusqu'à 8 atomes de carbone, R₃₄ représente un atome d'hydrogène, un radical alkyle renfermant de 1 à 3 atomes de carbone, un radical -C≡CH,
p) soit un groupement 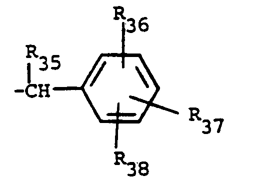 dans lequel R₃₅ représente un atome d'hydrogène, un radical alkyle renfermant de 1 à 3 atomes de carbone, un radical -C≡N, -C≡CH ou CF₃, R₃₆ et R₃₈, identiques ou différents l'un de l'autre, représentent un atome d'hydrogène, un radical alkyle renfermant de 1 à 3 atomes de carbone éventuellement substitué par un ou plusieurs atomes d'halogène, un radical alcényle renfermant de 2 à 4 atomes de carbone ou un atome d'halogène et R₃₇ représente un radical phényle éventuellement substitué par un ou plusieurs radicaux alkyle renfermant de 1 à 3 atomes de carbone ou par un ou plusieurs atomes d'halogène, R' représente un atome d'hydrogène, un radical alkyle renfermant de 1 à 18 atomes de carbone, linéaire, ramifié, saturé ou insaturé éventuellement substitué par un ou plusieurs groupements fonctionnels, identiques ou différents, ou R' représente un radical cycloaliphatique comportant de 3 à 7 atomes de carbone éventuellement substitué par un ou plusieurs groupements fonctionnels identiques ou différents, ou R' représente un groupement aryle renfermant de 6 à 14 atomes de carbone éventuellement substitué par un ou plusieurs groupements fonctionnels identiques ou différents, ou R' représente un radical hétérocyclique éventuellement substitué par un ou plusieurs groupements fonctionnels identiques ou différents, sous forme de mélanges d'isomères ou d'isomères séparés au niveau du cycle cyclopropane, caractérisé en ce que l'on traite un ester de formule (II) : 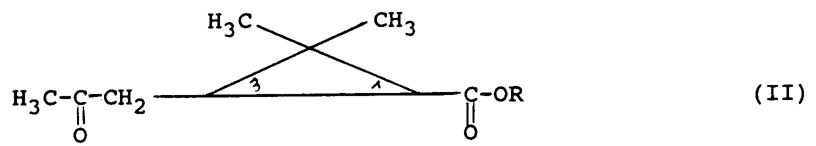 dans laquelle R est défini comme ci-dessus, par un agent d'halogénation, pour obtenir un composé de formule (III) : 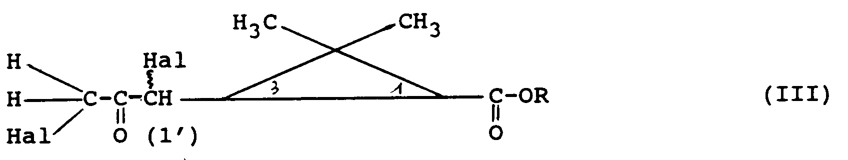 dans laquelle R est défini comme ci-dessus, et Hal représente un atome d'halogène, se présentant sous la forme d'un mélange d'isomères au niveau de l'atome de carbone en position 1', que l'on traite par un agent basique en présence d'un composé de formule R'-OH, dans laquelle R' est défini comme ci-dessus, pour obtenir le composé de formule (I) attendu.

2. Procédé selon la revendication 1, caractérisé en ce que l'agent d'halogénation est un agent de chloration ou de bromation.

3. Procédé selon la revendication 2, caractérisé en ce que l'agent d'halogénation est choisi dans le groupe constitué par le brome, le chlore, les N-bromo et N-chloro succinimides et acétamides, les perbromure et perchlorure de pyridinium et les perbromure et perchlorure d'hydrobromure et hydrochlorure de pyridinium.

4. Procédé selon l'une quelconque des revendications 1 à 3, caractérisé en ce que l'on utilise environ 2 équivalents d'agent d'halogénation.

5. Procédé selon l'une quelconque des revendications 1 à 4, caractérisé en ce que l'agent basique utilisé est choisi dans le groupe constitué par les hydrures, les alcoolates, les amidures, les carbonates alcalins et alcalino-terreux et les amines tertiaires.

6. Procédé selon l'une quelconque des revendications 1 à 5, caractérisé en ce que l'action de l'agent basique en présence d'un composé de formule R'-OH est effectué au sein d'un cosolvant approprié.

7. Procédé selon l'une quelconque des revendications 1 à 6, caractérisé en ce que R représente un reste d'ester clivable en milieu neutre ou acide, choisi dans le groupe constitué par les radicaux alkyle, linéaires ou ramifiés renfermant de 1 à 18 atomes de carbone, les radicaux alkyles linéaires ou ramifiés renfermant de 1 à 18 atomes de carbone substitués par un ou plusieurs atomes d'halogène, les radicaux alkyles renfermant de 1 à 4 atomes de carbone, substitués par un groupement silylé et les radicaux alkyles renfermant de 1 à 4 atomes de carbone substitués par un groupement O-alkyle, O-aryle ou O-aralkyle, alkyle renfermant de 1 à 4 atomes de carbone et aryle renfermant de 6 à 14 atomes de carbone.

8. Procédé selon la revendication 7, caractérisé en ce que R représente un reste d'ester clivable en milieu neutre ou acide, choisi dans le groupe constitué par les radicaux alkyles renfermant de 1 à 4 atomes de carbone, les radicaux alkyles renfermant de 1 à 4 atomes de carbone substitué par un ou plusieurs atomes de chlore ou de brome, les radicaux alkyles renfermant de 1 à 4 atomes de carbone substitués par un groupement alkylsilyle et les radicaux alkyles renfermant de 1 à 4 atomes de carbone substitués par un groupement O-alkyle, O-aryle ou O-aralkyle, tel que défini à la revendication 7.

9. Procédé selon la revendication 7 ou 8, caractérisé en ce que R représente un radical alkyle renfermant de 1 à 4 atomes de carbone, de préférence terbutyle, ou un radical méthyle ou éthyle substitué par un ou plusieurs atomes de chlore ou de brome, de préférence un radical bromo ou chloro méthyle ou un radical mono, di ou tribromo ou chloroéthyle.

10. Procédé selon l'une quelconque des revendications 1 à 6, caractérisé en ce que R représente un reste d'ester choisi dans le groupe constitué par :
- le radical benzyle substitué par un ou plusieurs atomes d'halogène,
- les radicaux de formule : 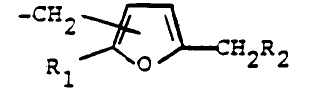 dans laquelle R₁ et R₂ sont définis comme précédemment,
- les radicaux de formule : 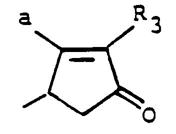 dans laquelle a et R₃ sont définis comme précédemment,
- les radicaux de formule : 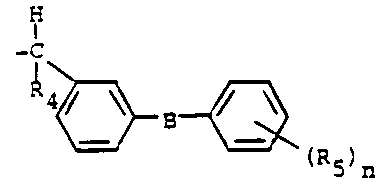 dans laquelle R₄, R₅ et n sont définis comme précédemment,
- les radicaux de formule : 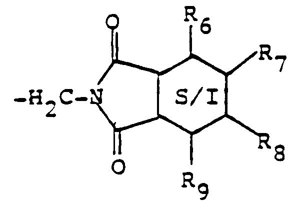 dans laquelle R₆, R₇, R₈, R₉ et S/I sont définis comme précédemment,
- les radicaux de formule : 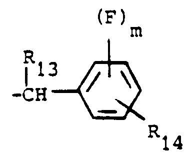 dans laquelle R₁₃, R₁₄ et m sont définis comme précédemment,
- les radicaux de formule : 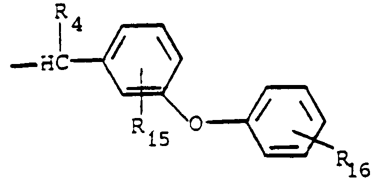 dans laquelle R₄, R₁₅ et R₁₆ sont définis comme précédemment,
- les radicaux de formule : 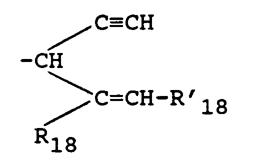 dans laquelle R₁₈ et R'₁₈ sont définis comme précédemment,
- les radicaux de formule : 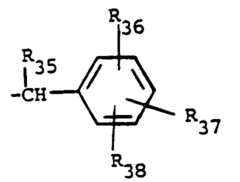 dans laquelle R₃₅, R₃₆, R₃₇ et R₃₈ sont définis comme précédemment.

11. Procédé selon la revendication 10, caractérisé en ce que R représente un reste d'ester choisi dans le groupe constitué par :
- le radical benzyle substitué par 1 à 5 atomes de fluor,
- les radicaux de formule : 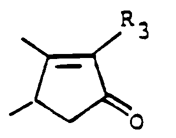 dans laquelle R₃ représente un radical -CH₂-CH-CH₂ ou -CH₂-C≡CH,
- les radicaux de formule : 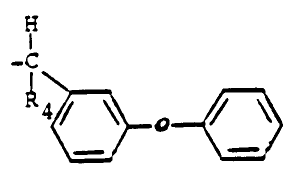 dans laquelle R₄ est défini comme précédemment,
- le radical de formule : 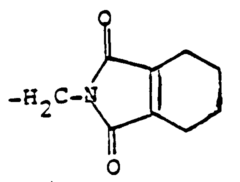
- les radicaux de formule : 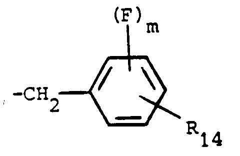 dans laquelle R₁₄ et m sont définis comme précédemment,
- les radicaux de formule : 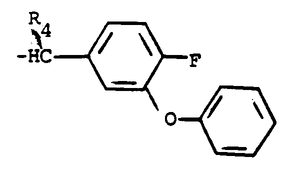 dans laquelle R₄ est défini comme précédemment,
- les radicaux de formule : 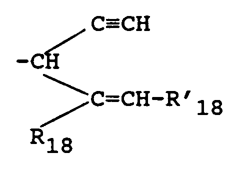 dans laquelle R₁₈ et R'₁₈ sont définis comme précédemment,
- les radicaux de formule : 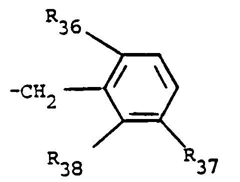 dans laquelle R₃₆ représente un atome d'hydrogène, un atome de fluor ou de chlore, R₃₇ représente un radical phényle ou 3-fluorophényle et R₃₈ représente un atome d'hydrogène, un atome de fluor ou de chlore, ou un radical méthyle.

12. Procédé selon l'une quelconque des revendications 1 à 11, caractérisé en ce que l'on utilise au départ un composé de formule R'-OH dans laquelle R' représente un atome d'hydrogène, un radical alkyle linéaire, ramifié ou cyclique renfermant de 1 à 8 atomes de carbone, et notamment un radical éthyle, un radical terbutyle ou encore un radical cyclopropyle ou cyclopropylméthyle, un radical alkyle linéaire ou ramifié renfermant de 1 à 8 atomes de carbone substitué par un ou plusieurs atomes d'halogène, et notamment par un ou plusieurs atomes de fluor, un radical (CH₂)ₘ-O-(CH₂)ₙ-CH₃ dans lequel m représente un nombre entier pouvant varier de 1 à 8 et n représente un nombre entier pouvant varier de 0 à 8, et notamment le radical -CH₂-O-CH₃.

13. Procédé selon l'une quelconque des revendications 1 à 6 et 10 à 12, caractérisé en ce que l'on utilise au départ un composé de formule (II) de structure (1R,cis) dans laquelle R représente un reste d'alcool (R,S) ou (S) α-cyano 3-phénoxybenzylique ou un reste d'alcool (R,S) ou (S) α-cyano 4-fluoro 3-phénoxybenzylique et un composé de formule R'-OH dans laquelle R' représente un radical méthyle, éthyle, terbutyle ou 1,1,1,3,3,3-hexafluoropropyle.

14. Composés de formule (I) telle que définie à la revendication 1, dans laquelle R représente un radical méthyle substitué par un atome de brome ou de chlore ou un radical éthyle substitué par 1, 2 ou 3 atomes de brome ou de chlore.

15. Composés de formule (III), tels que définis à la revendication 1, sous forme de mélanges d'isomères ou d'isomères séparés, au niveau du cycle cyclopropane.

16. Composés de formule (II), tels que définis à la revendication 1, sous forme de mélanges d'isomères ou d'isomères séparés, au niveau du cycle cyclopropane, à l'exception de ceux pour lesquels R représente un radical méthyle, éthyle, 3-phénoxybenzyle ou α-cyano 3-phénoxybenzyle.

## Claims

1. Preparation process for compounds of formula (I): 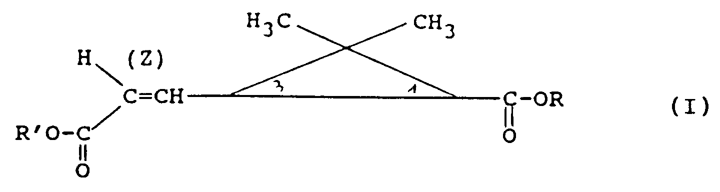 in which R represents either a remainder of a cleavable ester or a remainder of an ester known from the chemistry of the pyrethrinoids, namely:
a) either a benzyl radical optionally substituted on the aromatic vertices by one or more radicals chosen from the group constituted by alkyl radicals containing 1 to 4 carbon atoms, alkenyl radicals containing 2 to 6 carbon atoms, alkenyloxy radicals containing 2 to 6 carbon atoms, alkadienyl radicals containing 4 to 8 carbon atoms, the methylenedioxy radical and halogen atoms,
b) or a group 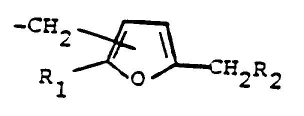 in which the substituent R₁ represents a hydrogen atom or a methyl radical and the substituent R₂ represents a monocyclic aryl radical or a -C≡CH group,
c) or a group 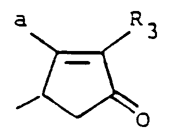 in which a represents a hydrogen atom or a methyl radical and R₃ represents an aliphatic organic radical containing 2 to 6 carbon atoms and one or more carbon-carbon unsaturations,
d) or a group 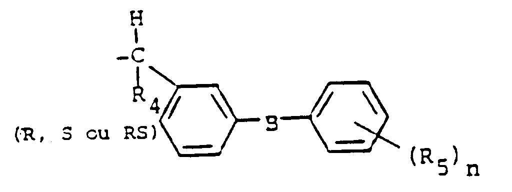 in which B represents an oxygen or sulphur atom or a 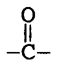 or -CH₂- group, R₄ represents a hydrogen atom, a chlorine, bromine or iodine atom, a -C≡N radical, a methyl radical, a CONH₂ radical, a -CSNH₂ radical or a -C≡CH radical, R₅ represents a halogen atom or a methyl radical and n represents a number equal to 0, 1 or 2,
e) or a group 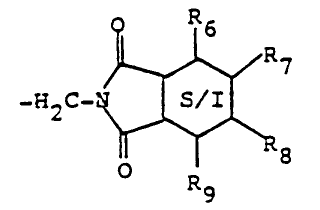 in which the substituents R₆, R₇, R₈ and R₉ represent a hydrogen atom, a chlorine atom or a methyl radical and in which S/I symbolizes an aromatic ring or an analogous dihydro, tetrahydro or hexahydro ring,
f) or a (succimido or maleimido) methylene group,
g) or a group 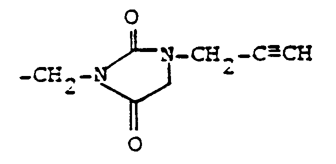
h) or a group 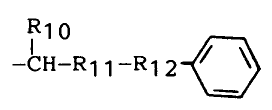 in which R₁₀ represents a hydrogen atom or a CN radical, R₁₂ represents a -CH₂- radical or an oxygen atom, R₁₁ represents a thiazolyl or thiadiazolyl radical whose bond with 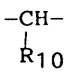 can be found in one any of the available positions, R₁₂ being linked to R₁₁ by the carbon atom contained between the sulphur atom and the nitrogen atom,
i) or a group 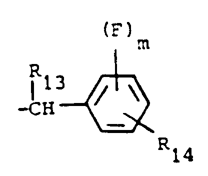 in which R₁₃ represents a hydrogen atom or a -CN or -C≡CH radical, R₁₄ represents a trifluoromethyl radical or an alkyl, alkenyl or alkynyl radical containing at most 6 carbon atoms and m represents a number equal to 1, 2, 3 or 4,
j) or a group 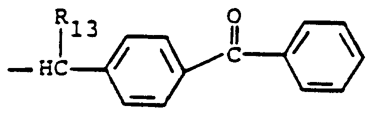 in which R₁₃ is defined as above,
k) or a group 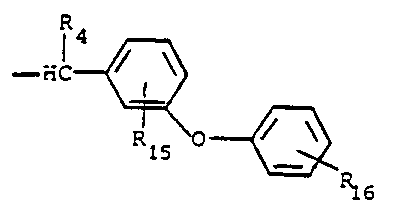 in which R₄ is defined as above, R₁₅ represents a fluorine, chlorine or bromine atom and R₁₆ represents a hydrogen, fluorine, chlorine or bromine atom,
l) or a group 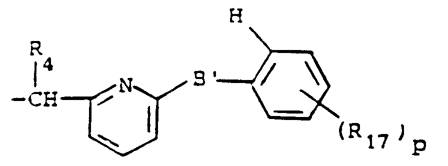 in which R₄ is defined as above, each of the R₁₇'s represents independently one of the following groups: alkyl containing 1 to 4 carbon atoms, alkoxy containing 1 to 4 carbon atoms, alkylthio containing 1 to 4 carbon atoms, alkylsulphonyl containing 1 to 4 carbon atoms, trifluoromethyl, 3,4-methylenedioxy, chloro, fluoro or bromo, p represents a number equal to 0, 1 or 2 and B' represents an oxygen atom or a sulphur atom,
m) or a group 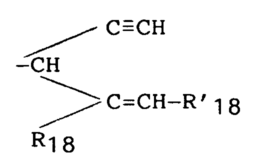 in which R₁₈ represents a fluorine atom or a methyl radical and R'₁₈ represents a methyl, ethyl or propargyl radical,
n) or a group 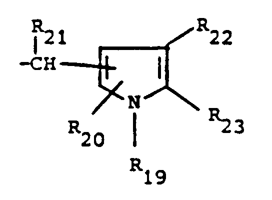 in which R₂₁ represents a hydrogen atom, a -C≡N, -C≡CH, CF₃ group or an alkyl radical containing 1 to 3 carbon atoms, R₂₀, R₂₂ and R₂₃ identical to or different from each other, represent a hydrogen atom, a halogen atom, an alkyl radical containing up to 18 carbon atoms, an aryl radical containing up to 14 carbon atoms, an aralkyl radical containing up to 18 carbon atoms, a cyano radical, a -CF₃ radical, a -CO₂-alkyl radical containing up to 8 carbon atoms, an NO₂ radical, an alkoxy radical containing up to 8 carbon atoms, an 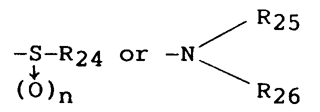 radical, n being equal to 0, 1 or 2 and the R₂₄, R₂₅ and R₂₆ radicals representing an alkyl radical containing 1 to 8 carbon atoms, the R₂₂ and R₂₃ radicals being able to form a saturated or unsaturated carbonaceous homocycle containing up to 8 carbon atoms and R₁₉ represents
- either a 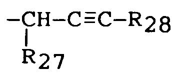 radical in which R₂₇ and R₂₈, identical to or different from each other, represent a hydrogen atom, a halogen atom, an alkyl radical containing 1 to 8 carbon atoms or an aryl radical containing up to 14 carbon atoms
- or a 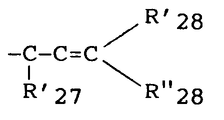 radical, in which R'₂₇, R'₂₈ and R''₂₈, identical to or different from each other, represent one of the values indicated above for R₂₇ and R₂₈, the dotted lines represent an optional second bond,
- or a 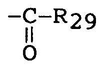 radical in which R₂₉ can have the values indicated previously for R₂₂ and R₂₃, with the exception of halogen, cyano, NO₂, 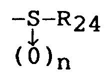 in which n is equal to 1 or 2 and 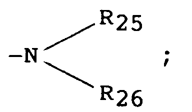
- or a 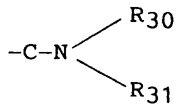 radical, in which R₃₀ and R₃₁, identical to or different from each other, represent a hydrogen atom, an alkyl radical containing 1 to 18 carbon atoms, an aryl radical containing up to 14 carbon atoms, an aralkyl radical containing up to 18 carbon atoms, a CF₃ radical, a CO₂-alkyl radical containing up to 8 carbon atoms or an alkoxy radical containing up to 8 carbon atoms,
o) or a group 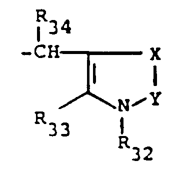 in which X represents a sulphur or oxygen atom, Y represents a >C=O, >C=S or >CH₂ group, R₃₂ represents a hydrogen atom, a saturated or unsaturated, linear, branched or cyclic alkyl radical containing up to 8 carbon atoms, optionally substituted by one or more halogens, or an aryl radical containing up to 14 carbon atoms, R₃₃ represents a hydrogen atom, a saturated or unsaturated, linear, branched or cyclic alkyl radical containing up to 8 carbon atoms, optionally substituted by one or more halogens, or R₃₃ represents an aryl radical containing up to 14 carbon atoms, a CF₃, -NO₂, -C≡N radical, a halogen atom, an alkoxyl radical containing up to 8 carbon atoms or a -CO₂-alkyl radical containing up to 8 carbon atoms, R₃₄ represents a hydrogen atom, an alkyl radical containing 1 to 3 carbon atoms, a -C≡CH radical,
p) or a group 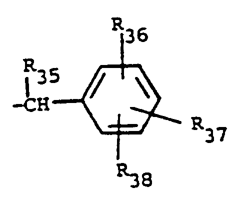 in which R₃₅ represents a hydrogen atom, an alkyl radical containing 1 to 3 carbon atoms, a -C≡N, -C≡CH or CF₃ radical, R₃₆ and R₃₈, identical to or different from each other, represent a hydrogen atom, an alkyl radical containing 1 to 3 carbon atoms optionally substituted by one or more halogen atoms, an alkenyl radical containing 2 to 4 carbon atoms or a halogen atom and R₃₇ represents a phenyl radical optionally substituted by one or more alkyl radicals containing 1 to 3 carbon atoms or by one or more halogen atoms, R' represents a hydrogen atom, a saturated or unsaturated, linear, branched alkyl radical containing 1 to 18 carbon atoms, optionally substituted by one or more identical or different functional groups, or R' represents a cycloaliphatic radical containing 3 to 7 carbon atoms optionally substituted by one or more identical or different functional groups, or R' represents an aryl group containing 6 to 14 carbon atoms optionally substituted by one or more identical or different functional groups, or R' represents a heterocyclic radical optionally substituted by one or more identical or different functional groups, in the form of mixtures of isomers or separate isomers at the level of the cyclopropane ring, characterized in that an ester of formula (II): 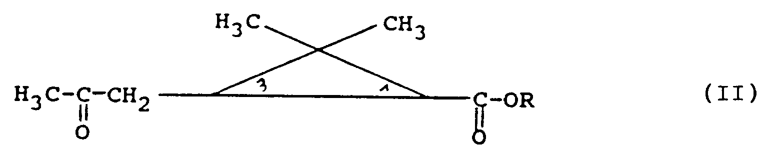 in which R is defined as above, is treated with a halogenation agent, in order to obtain a compound of formula (III): 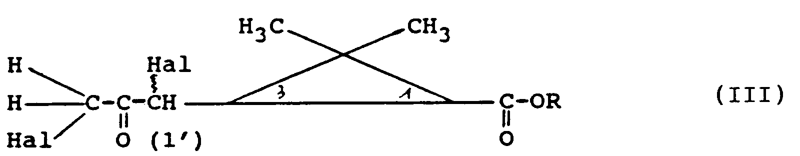 in which R is defined as above, and Hal represents a halogen atom, being in the form of a mixture of isomers at the level of the carbon atom in position 1', which is treated with a basic agent in the presence of a compound of formula R'-OH, in which R' is defined as above, in order to obtain the expected compound of formula (I).

2. Process according to claim 1, characterized in that the halogenation agent is a chlorination or bromination agent.

3. Process according to claim 2, characterized in that the halogenation agent is chosen from the group constituted by bromine, chlorine, N-bromo and N-chloro succinimides and acetamides, pyridinium perbromide and perchloride and pyridinium hydrobromide and hydrochloride perbromide and perchloride.

4. Process according to any one of claims 1 to 3, characterized in that about 2 equivalents of the halogenation agent are used.

5. Process according to any one of claims 1 to 4, characterized in that the basic agent used is chosen from the group constituted by hydrides, alcoholates, amides, alkali and alkaline-earth carbonates and tertiary amines.

6. Process according to any one of claims 1 to 5, characterized in that the action of the basic agent in the presence of a compound of formula R'-OH is carried out in an appropriate cosolvant.

7. Process according to any one of claims 1 to 6, characterized in that R represents a cleavable ester remainder in neutral or acid medium, chosen from the group constituted by linear or branched alkyl radicals containing 1 to 18 carbon atoms, linear or branched alkyl radicals containing 1 to 18 carbon atoms substituted by one or more halogen atoms, alkyl radicals containing 1 to 4 carbon atoms, substituted by a silylated group, and alkyl radicals containing 1 to 4 carbon atoms substituted by an O-alkyl, O-aryl or O-aralkyl group, alkyl radicals containing 1 to 4 carbon atoms and aryl radicals containing 6 to 14 carbon atoms.

8. Process according to claim 7, characterized in that R represents a cleavable ester remainder in neutral or acid medium, chosen from the group constituted by alkyl radicals containing 1 to 4 carbon atoms, alkyl radicals containing 1 to 4 carbon atoms substituted by one or more chlorine or bromine atoms, alkyl radicals containing 1 to 4 carbon atoms substituted by an alkylsilyl group and alkyl radicals containing 1 to 4 carbon atoms substituted by an O-alkyl, O-aryl or O-aralkyl group, as defined in claim 7.

9. Process according to claim 7 or 8, characterized in that R represents an alkyl radical containing 1 to 4 carbon atoms, preferably terbutyl, or a methyl or ethyl radical substituted by one or more chlorine or bromine atoms, preferably a bromo or chloro methyl radical or a mono-, di- or tribromo or chloroethyl radical.

10. Process according to any one of claims 1 to 6, characterized in that R represents an ester remainder chosen from the group constituted by:
- the benzyl radical substituted by one or more halogen atoms,
- the radicals of formula: 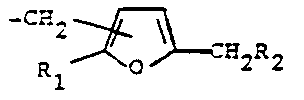 in which R₁ and R₂ are defined as previously,
- the radicals of formula: 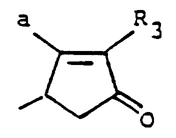 in which a and R₃ are defined as previously,
- the radicals of formula: 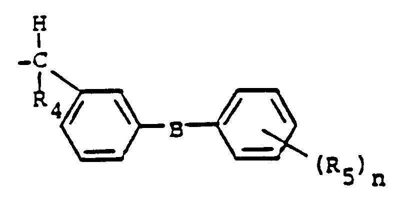 in which R₄, R₅ and n are defined as previously,
- the radicals of formula: 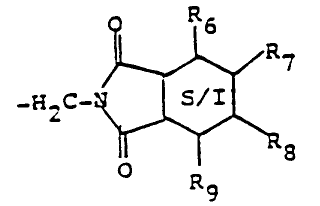 in which R₆, R₇, R₈, R₉ and S/I are defined as previously,
- the radicals of formula: 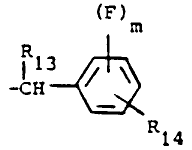 in which R₁₃, R₁₄ and m are defined as previously,
- the radicals of formula: 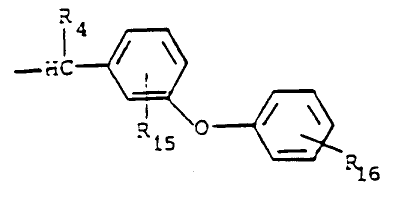 in which R₄, R₁₅ and R₁₆ are defined as previously,
- the radicals of formula: 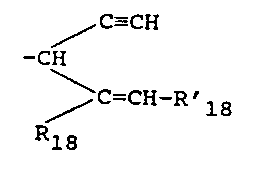 in which R₁₈ and R'₁₈ are defined as previously,
- the radicals of formula: 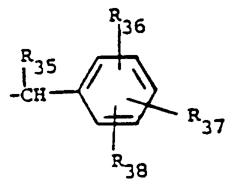 in which R₃₅, R₃₆, R₃₇ and R₃₈ are defined as previously.

11. Process according to claim 10, characterized in that R represents an ester remainder chosen from the group constituted by:
- the benzyl radical substituted by 1 to 5 fluorine atoms,
- the radicals of formula: 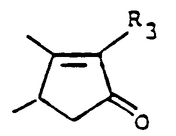 in which R₃ represents a -CH₂-CH-CH₂ or -CH₂-C≡CH radical,
- the radicals of formula: 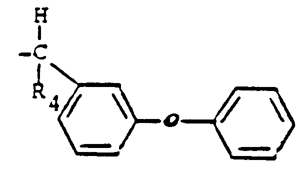 in which R₄ is defined as previously,
- the radical of formula: 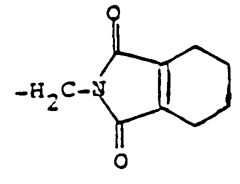
- the radicals of formula: 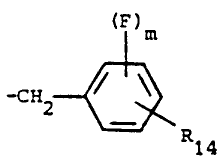 in which R₁₄ and m are defined as previously,
- the radicals of formula: 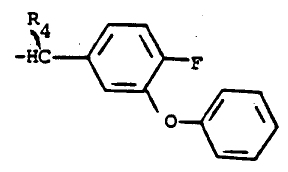 in which R₄ is defined as previously,
- the radicals of formula: 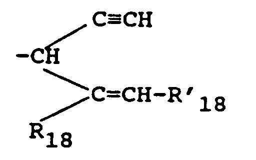 in which R₁₈ and R'₁₈ are defined as previously,
- the radicals of formula: 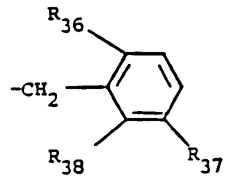 in which R₃₆ represents a hydrogen atom, a fluorine or chlorine atom, R₃₇ represents a phenyl or 3-fluorophenyl radical and R₃₈ represents a hydrogen atom, a fluorine or chlorine atom, or a methyl radical.

12. Process according to any one of claims 1 to 11, characterized in that a compound of formula R'-OH is used at the start in which R' represents a hydrogen atom, a linear, branched or cyclic alkyl radical containing 1 to 8 carbon atoms, and in particular an ethyl radical, a terbutyl radical or also a cyclopropyl or cyclopropylmethyl radical, a linear or branched alkyl radical containing 1 to 8 carbon atoms substituted by one or more halogen atoms, and in particular by one or more fluorine atoms, a (CH₂)ₘ-O-(CH₂)ₙ-CH₃ radical in which m represents an integer which can vary from 1 to 8 and n represents an integer which can vary from 0 to 8, and in particular the -CH₂-O-CH₃ radical.

13. Process according to any one of claims 1 to 6 and 10 to 12, characterized in that a compound of formula (II) of (1R,cis) structure in which R represents a remainder of (R,S) or (S) alpha-cyano 3-phenoxybenzyl alcohol or a remainder of (R,S) or (S) alpha-cyano 4-fluoro 3-phenoxybenzyl alcohol and a compound of formula R'-OH in which R' represents a methyl, ethyl, terbutyl or 1,1,1,3,3,3-hexafluoropropyl radical are used at the start.

14. Compounds of formula (I) as defined in claim 1, in which R represents a methyl radical substituted by a bromine or chlorine atom or an ethyl radical substituted by 1, 2 or 3 bromine or chlorine atoms.

15. Compounds of formula (III), as defined in claim 1, in the form of isomer mixtures or separate isomers, at the level of the cyclopropane ring.

16. Compounds of formula (II), as defined in claim 1, in the form of isomer mixtures or separate isomers, at the level of the cyclopropane ring, with the exception of those in which R represents a methyl, ethyl, 3-phenoxybenzyl or alphacyano 3-phenoxybenzyl radical.

## Patentansprüche

1. Verfahren zur Herstellung von Verbindungen der Formel (I) 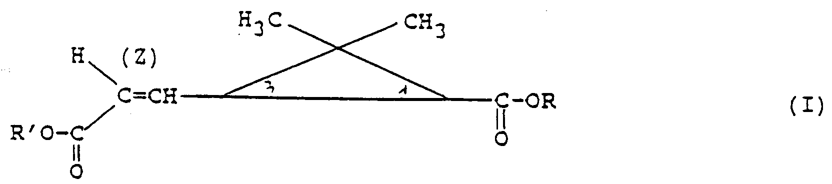 worin R einen spaltbaren Esterrest oder einen in der Chemie der Pyrethrinoide bekannten Esterrest bedeutet, nämlich
a) entweder einen Benzylrest, gegebenenfalls substituiert an den aromatischen Spitzen durch einen oder mehrere Reste, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxyrest und den Halogenatomen,
b) oder eine Gruppe 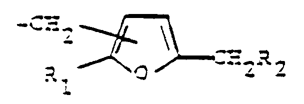 worin der Substituent R₁ ein Wasserstoffatom oder einen Methylrest darstellt und der Substituent R₂ einen monocyclischen Arylrest oder eine Gruppe -C≡CH bedeutet,
c) oder eine Gruppe 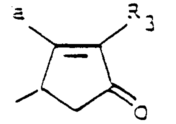 worin a ein Wasserstoffatom oder einen Methylrest bedeutet und R₃ einen aliphatischen, organischen Rest mit 2 bis 6 Kohlenstoffatomen und einer oder mehreren Kohlenstoff-Kohlenstoff-Unsättigungen darstellt,
d) oder eine Gruppe 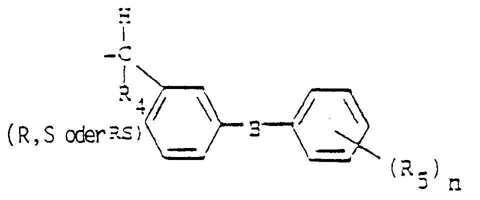 worin B für ein Sauerstoff- oder Schwefelatom oder eine Gruppe 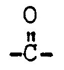 oder -CH₂- steht, R₄ ein Wasserstoffatom, ein Chlor-, Brom- oder Jodatom, einen Rest -C≡N, eine Methylgruppe, einen Rest -CONH₂, einen Rest -CSNH₂ oder einen Rest -C≡CH wiedergibt, R₅ ein Halogenatom oder einen Methylrest darstellt und n für eine Zahl entsprechend 0, 1 oder 2 steht,
e) oder eine Gruppe 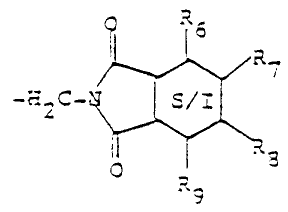 worin die Substituenten R₆, R₇, R₈ und R₉ ein Wasserstoffatom, ein Chloratom oder eine Methylgruppe bedeuten und worin S/I einen aromatischen Ring oder einen analogen Dihydro-, Tetrahydro- oder Hexahydroring symbolisiert,
f) oder eine(Succinimido- oder Maleimido-) methylengruppe,
g) oder eine Gruppe 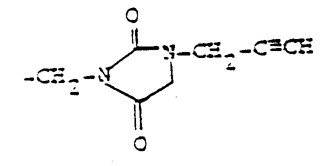
h) oder eine Gruppe 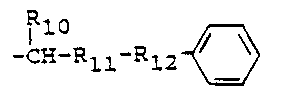 worin R₁₀ fur ein Wasserstoffatom oder einen Rest CN steht, R₁₂ einen Rest -CH₂- oder ein Sauerstoffatom wiedergibt, R₁₁ einen Thiazolyl- oder Thiadiazolylrest, dessen Bindung mit 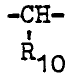 sich in irgendeiner der verfügbaren Positionen befinden kann, bedeutet, wobei R₁₂ an R₁₁ über das zwischen dem Schwefelatom und einem Sauerstoffatom befindliche Kohlenstoffatom gebunden ist,
i) oder eine Gruppe 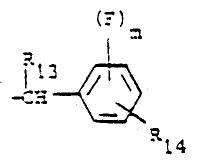 worin R₁₃ ein Wasserstoffatom oder einen Rest -CN oder -C≡CH darstellt, R₁₄ einen Trifluormethylrest oder einen Alkyl-, Alkenyl- oder Alkinylrest mit höchstens 6 Kohlenstoffatomen bedeutet und m für eine Zahl entsprechend 1, 2, 3 oder 4 steht,
j) oder eine Gruppe 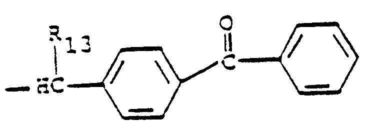 worin R₁₃ wie vorstehend definiert ist,
k) oder eine Gruppe 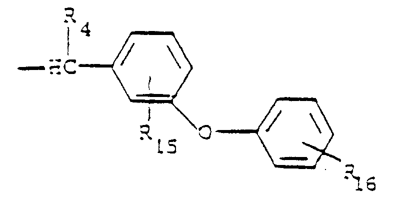 worin R₄ wie vorstehend definiert ist, R₁₅ ein Fluor-, Chlor- oder Bromatom bedeutet und R₁₆ ein Wasserstoff-, Fluor-, Chlor oder Bromatom wiedergibt,
l) oder eine Gruppe 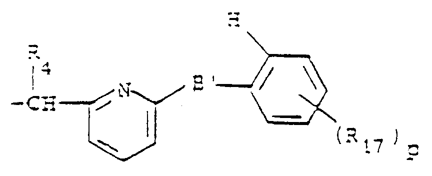 worin R₄ wie vorstehend definiert ist, eine jede der Gruppen R₁₇ unabhängig eine Alkylgruppe mit 1 bis 4 Kohlenstoffatomen, Alkoxy mit 1 bis 4 Kohlenstoffatomen, Alkylthio mit 1 bis 4 Kohlenstoffatomen, Alkylsulfonyl mit 1 bis 4 Kohlenstoffatomen, Trifluormethyl, 3,4-Methylendioxy, Chlor, Fluor oder Brom bedeutet, p für eine Zahl entsprechend 0, 1 oder 2 steht und B' ein Sauerstoff-oder Schwefelatom darstellt,
m) oder eine Gruppe 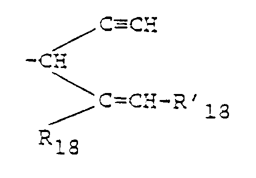 worin R₁₈ für ein Fluoratom oder eine Methylgruppe steht und R'₁₈ einen Methyl-, Ethyl- oder Propargylrest bedeutet,
n) oder eine Gruppe 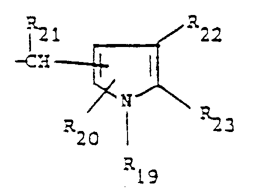 worin R₂₁ ein Wasserstoffatom, eine Gruppe -C≡N, -C≡CH, -CF₃ oder einen Alkylrest mit 1 bis 3 Kohlenstoffatomen darstellt, R₂₀, R₂₂ und R₂₃,identisch oder verschieden voneinander, ein Wasserstoffatom, ein Halogenatom, einen Alkylrest mit bis zu 18 Kohlenstoffatomen, einen Arylrest mit bis zu 14 Kohlenstoffatomen, einen Aralkylrest mit bis zu 18 Kohlenstoffatomen, eine Cyanogruppe, einen Rest -CF₃, einen Rest -CO₂-alkyl mit bis zu 8 Kohlenstoffatomen, eine Gruppe NO₂, einen Alkoxyrest mit bis zu 8 Kohlenstoffatomen, einen Rest 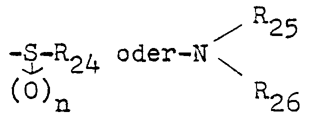 bedeuten, wobei n für 0, 1 oder 2 steht und die Reste R₂₄, R₂₅ und R₂₆ einen Alkylrest mit 1 bis 8 Kohlenstoffatomen bedeuten, die Reste R₂₂ und R₂₃ einen gesättigten oder ungesättigten Kohlenstoff-Homocyclus mit bis zu 8 Kohlenstoffatomen bilden können und R₁₉ bedeutet:
entweder einen Rest 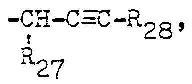 worin R₂₇ und R₂₈,identisch oder verschieden voneinander, ein Wasserstoffatom, ein Halogenatom, einen Alkylrest mit 1 bis 8 Kohlenstoffatomen, einen Arylrest mit bis zu 14 Kohlenstoffatomen bedeuten,
oder einen Rest 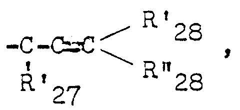 worin R'₂₇, R'₂₈ und R''₂₈, identisch oder verschieden voneinander, eine der vorstehend für R₂₇ und R₂₈ angegebenen Bedeutungen besitzen, die gestrichelten Linien eine etwaige zweite Bindung darstellen,
oder einen Rest 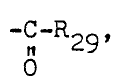 worin R₂₉ die vorstehend fur R₂₂ und R₂₃ angegebenen Bedeutungen haben kann, ausgenommen Halogen, Cyano, NO₂, 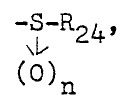 worin n fur 1 oder 2 steht, und 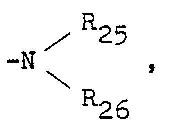 oder einen Rest 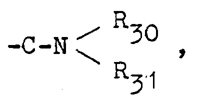 worin R₃₀ und R₃₁, identisch oder voneinander verschieden, ein Wasserstoffatom, einen Alkylrest mit 1 bis 18 Kohlenstoffatomen, einen Arylrest mit bis zu 14 Kohlenstoffatomen, einen Aralkylrest mit bis zu 18 Kohlenstoffatomen, einen Rest CF₃, einen Rest CO₂-alkyl mit bis zu 8 Kohlenstoffatomen oder einen Alkoxyrest mit bis zu 8 Kohlenstoffatomen bedeuten,
o) oder eine Gruppe 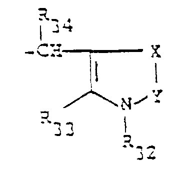 worin X ein Schwefel- oder Sauerstoffatom darstellt, Y eine Gruppe >C=O, >C=S oder >CH₂ bedeutet, R₃₂ ein Wasserstoffatom, einen gesättigten oder ungesättigten, linearen, verzweigten oder cyclischen Alkylrest mit bis zu 8 Kohlenstoffatomen, welcher gegebenenfalls substituiert ist durch ein oder mehrere Halogene, oder einen Arylrest mit bis zu 14 Kohlenstoffatomen bedeutet, R₃₃ ein Wasserstoffatom, einen gesättigten oder ungesättigten, linearen, verzweigten oder cyclischen Alkylrest mit bis zu 8 Kohlenstoffatomen, gegebenenfalls substituiert durch ein oder mehrere Halogene,wiedergibt, oder R₃₃ einen Arylrest mit bis zu 14 Kohlenstoffatomen, einen Rest -CF₃, -NO₂, -C≡N, ein Halogenatom, einen Alkoxyrest mit bis zu 8 Kohlenstoffatomen oder einen Rest -CO₂-alkyl mit bis zu 8 Kohlenstoffatomen bedeutet, R₃₄ ein Wasserstoffatom, einen Alkylrest mit 1 bis 3 Kohlenstoffatomen, einen Rest -C≡CH wiedergibt,
p) oder eine Gruppe 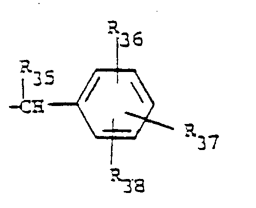 worin R₃₅ ein Wasserstoffatom, einen Alkylrest mit 1 bis 3 Kohlenstoffatomen, einen Rest -C≡N, -C≡CH oder CF₃ darstellt, R₃₆ und R₃₈, identisch oder verschieden voneinander, ein Wasserstoffatom, einen Alkylrest mit 1 bis 3 Kohlenstoffatomen, gegebenenfalls substituiert durch ein oder mehrere Halogenatome, einen Alkenylrest mit 2 bis 4 Kohlenstoffatomen oder ein Halogenatom bedeutet und R₃₇ einen Phenylrest, gegebenenfalls substituiert durch einen oder mehrere Alkylreste mit 1 bis 3 Kohlenstoffatomen oder durch ein oder mehrere Halogenatome, bedeutet, R' für ein Wasserstoffatom, einen gesättigten oder ungesättigten, linearen oder verzweigten Alkylrest mit 1 bis 18 Kohlenstoffatomen, gegebenenfalls substituiert durch eine oder mehrere funktionelle Gruppen, welche identisch oder voneinander verschieden sind, steht, oder R' einen cycloaliphatischen Rest mit 3 bis 7 Kohlenstoffatomen, gegebenenfalls substituiert durch eine oder mehrere, identische oder verschiedene funktionelle Gruppen, bedeutet oder R' eine Arylgruppe mit 6 bis 14 Kohlenstoffatomen, gegebenenfalls substituiert durch eine oder mehrere, identische oder verschiedene funktionelle Gruppen,bedeutet oder R' einen heterocyclischen Rest, gegebenenfalls substituiert durch eine oder mehrere identische oder verschiedene funktionelle Gruppen, bedeutet, in Form von Gemischen der Isomeren oder von getrennten Isomeren, im Hinblick auf den Cyclopropanring,
dadurch gekennzeichnet, daß man einen Ester der Formel (II) 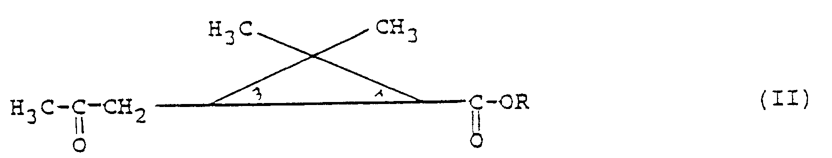 worin R wie vorstehend definiert ist, mit einem Halogenierungsmittel behandelt, um zu einer Verbindung der Formel (III) 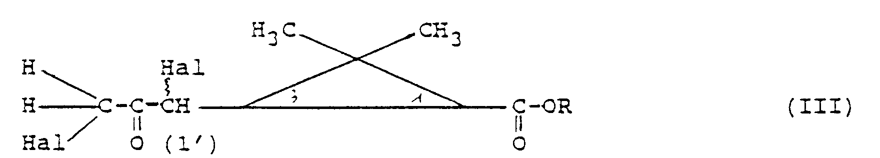 worin R wie vorstehend definiert ist und Hal für ein Halogenatom steht und die in Form eines Gemisches der Isomeren im Hinblick auf das Kohlenstoffatom in 1'-Stellung vorliegt, zu gelangen, welche man mit einem basischen Mittel in Anwesenheit einer Verbindung der Formel R'-OH, worin R' wie vorstehend definiert ist, behandelt, um zu der erwarteten Verbindung der Formel (I) zu gelangen.

2. Verfahren gemäß Anspruch 1, dadurch gekennzeichnet, daß das Halogenierungsmittel ein Chlorierungs- oder Bromierungsmittel ist.

3. Verfahren gemäß Anspruch 2, dadurch gekennzeichnet, daß das Halogenierungsmittel unter Brom, Chlor, N-Brom- und N-Chlor-succinimiden und -acetamiden, dem Pyridinium-perbromid und -perchlorid und dem Perbromid und Perchlorid von Pyridiniumhydrochlorid und -hydrobromid ausgewählt wird.

4. Verfahren gemäß einem der Ansprüche 1 bis 3, dadurch gekennzeichnet, daß man etwa 2 Äquivalente Halogenierungsmittel verwendet.

5. Verfahren gemäß einem der Ansprüche 1 bis 4, dadurch gekennzeichnet, daß das verwendete basische Mittel unter den Alkali- und Erdalkali-hydriden, -alkoholaten, -amiden, -carbonaten und den tertiären Aminen ausgewählt wird.

6. Verfahren gemäß einem der Ansprüche 1 bis 5, dadurch gekennzeichnet, daß die Umsetzung mit dem basischen Mittel in Anwesenheit einer Verbindung der Formel R'-OH in einem geeigneten Co-Lösungsmittel durchgeführt wird.

7. Verfahren gemäß einem der Ansprüche 1 bis 6, dadurch gekennzeichnet, daß R für einen in neutralem oder saurem Milieu spaltbaren Esterrest steht, ausgewählt unter den linearen oder verzweigten Alkylresten mit 1 bis 18 Kohlenstoffatomen, den linearen oder verzweigten Alkylresten mit 1 bis 18 Kohlenstoffatomen, substituiert durch ein oder mehrere Halogenatome, den Alkylresten mit 1 bis 4 Kohlenstoffatomen, substituiert durch eine Silylgruppe, und den Alkylresten mit 1 bis 4 Kohlenstoffatomen, substituiert durch eine O-Alkyl-, O-Aryl- oder O-Aralkylgruppe, wobei Alkyl 1 bis 4 Kohlenstoffatome und Aryl 6 bis 14 Kohlenstoffatome aufweist.

8. Verfahren gemäß Anspruch 7, dadurch gekennzeichnet, daß R einen in neutralem oder saurem Milieu spaltbaren Esterrest darstellt, ausgewählt unter den Alkylresten mit 1 bis 4 Kohlenstoffatomen, den Alkylresten mit 1 bis 4 Kohlenstoffatomen, substituiert durch ein oder mehrere Chlor- oder Bromatome, den Alkylresten mit 1 bis 4 Kohlenstoffatomen, substituiert durch eine Alkylsilylgruppe, und den Alkylresten mit 1 bis 4 Kohlenstoffatomen, substituiert durch eine O-Alkyl-, O-Aryl- oder O-Aralkylgruppe, wie in Anspruch 7 definiert.

9. Verfahren gemäß Anspruch 7 oder 8, dadurch gekennzeichnet, daß R einen Alkylrest mit 1 bis 4 Kohlenstoffatomen, bevorzugt tert.-Butyl, oder einen Methyl- oder Ethylrest, substituiert durch ein oder mehrere Chlor- oder Bromatome, bevorzugt einen Brom- oder Chlormethylrest oder einen Mono-, Di- oder Tribrom- oder -chlorethylrest,bedeutet.

10. Verfahren gemäß einem der Ansprüche 1 bis 6, dadurch gekennzeichnet, daß R einen Esterrest bedeutet, ausgewählt unter:
- dem Benzylrest, substituiert durch ein oder mehrere Halogenatome,
- den Resten der Formel 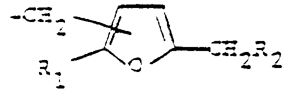 worin R₁ und R₂ wie vorstehend definiert sind,
- den Resten der Formel 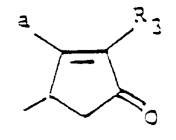 worin a und R₃ wie vorstehend definiert sind,
- den Resten der Formel 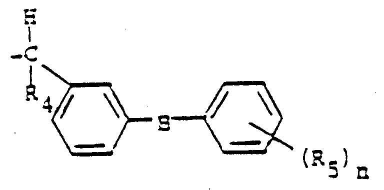 worin R₄, R₅ und n wie vorstehend definiert sind,
- den Resten der Formel 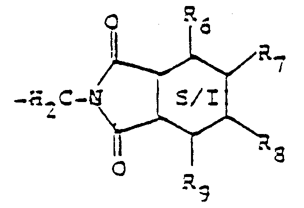 worin R₆, R₇, R₈, R₉ und S/I wie vorstehend definiert sind,
- den Resten der Formel 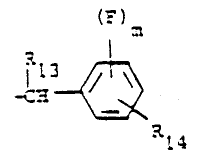 worin R₁₃, R₁₄ und m wie vorstehend definiert sind,
- den Resten der Formel 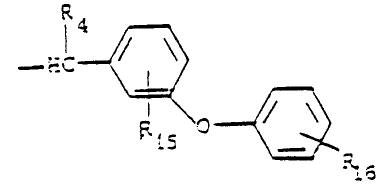 worin R₄, R₁₅ und R₁₆ wie vorstehend definiert sind,
- den Resten der Formel 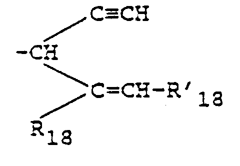 worin R₁₈ und R'₁₈ wie vorstehend definiert sind,
- den Resten der Formel 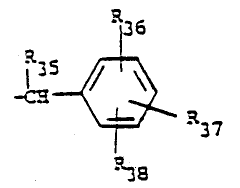 worin R₃₅, R₃₆, R₃₇ und R₃₈ wie vorstehend definiert sind.

11. Verfahren gemäß Anspruch 10, dadurch gekennzeichnet, daß R einen Esterrest bedeutet, ausgewählt unter:
- dem Benzylrest, substituiert durch 1 bis 5 Fluoratome,
- den Resten der Formel 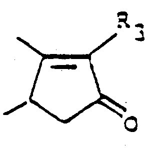 worin R₃ für einen Rest -CH₂-CH-CH₂ oder -CH₂-C≡CH steht,
- den Resten der Formel 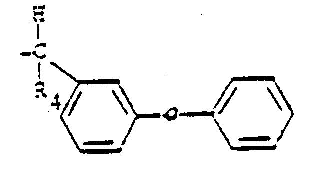 worin R₄ wie vorstehend definiert ist,
- dem Rest der Formel 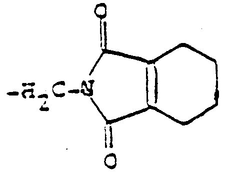
- den Resten der Formel 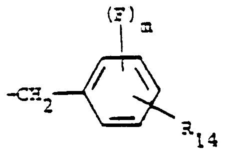 worin R₁₄ und m wie vorstehend definiert sind,
- den Resten der Formel 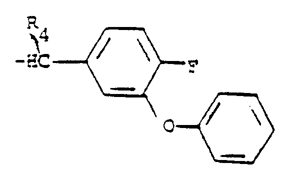 worin R₄ wie vorstehend definiert ist,
- den Resten der Formel 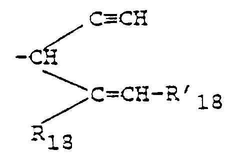 worin R₁₈ und R'₁₈ wie vorstehend definiert sind,
- den Resten der Formel 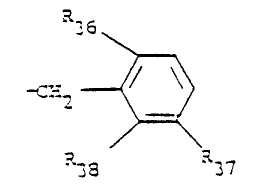 worin R₃₆ ein Wasserstoffatom, ein Fluor- oder Chloratom bedeutet, R₃₇ einen Phenyl- oder 3-Fluorphenylrest bedeutet und R₃₈ ein Wasserstoffatom, ein Fluor- oder Chloratom oder einen Methylrest wiedergibt.

12. Verfahren gemäß einem der Ansprüche 1 bis 11, dadurch gekennzeichnet, daß man von einer Verbindung der Formel R'-OH ausgeht, worin R' für ein Wasserstoffatom, einen linearen, verzweigten oder cyclischen Alkylrest mit 1 bis 8 Kohlenstoffatomen und insbesondere einen Ethylrest, einen tert.-Butylrest oder auch einen Cyclopropyl- oder Cyclopropylmethylrest, einen linearen oder verzweigten Alkylrest mit 1 bis 8 Kohlenstoffatomen, substituiert durch ein oder mehrere Halogenatome und insbesondere durch ein oder mehrere Fluoratome, einen Rest (CH₂)ₘ-O-(CH₂)ₙ-CH₃, worin m für eine ganze Zahl, variierend von 1 bis 8, und n für eine ganze Zahl, variierend von 0 bis 8, steht, und insbesondere den Rest -CH₂-O-CH₃ steht.

13. Verfahren gemäß einem der Ansprüche 1 bis 6 und 10 bis 12, dadurch gekennzeichnet, daß man von einer Verbindung der Formel (II) mit (1R,cis)-Struktur, worin R für einen (R,S)- oder (S)-α-Cyano-3-phenoxy-benzylalkoholrest oder einen (R,S)- oder (S)-α-Cyano-4-fluor-3-phenoxybenzylalkoholrest steht, und einer Verbindung der Formel R'-OH, worin R' für einen Methyl-, Ethyl-, tert.-Butyl- oder 1,1,1,3,3,3-Hexafluorpropylrest steht, ausgeht.

14. Verbindungen der Formel (I), wie in Anspruch 1 definiert, worin R für einen Methylrest, substituiert durch ein Brom- oder Chloratom, oder einen Ethylrest, substituiert durch 1, 2 oder 3 Brom- oder Chloratome, steht.

15. Verbindungen der Formel (III), wie in Anspruch 1 definiert, in Form von Gemischen der Isomeren oder getrennten Isomeren, im Hinblick auf den Cyclopropanring,

16. Verbindungen der Formel (II), wie in Anspruch 1 definiert, in Form von Gemischen der Isomeren oder getrennten Isomeren, im Hinblick auf den Cyclopropanring, ausgenommen diejenigen, worin R für einen Methyl-, Ethyl-, 3-Phenoxybenzyl- oder α-Cyano-3-phenoxybenzylrest steht.
